# EUROPEAN PATENT SPECIFICATION

(11) **EP 1 463 487 B1**
(45) Date of publication and mention of the grant of the patent: **19.05.2010**
(21) Application number: 02797447.6
(22) Date of filing: 19.12.2002
(51) Int. Cl.: A61K 9/127, A61K 31/355, A61K 45/06, A61P 35/00

(54) **LIPOSOMAL DELIVERY OF VITAMIN E BASED COMPOUNDS**
LIPOSOMALE ABGABE VON VERBINDUNGEN AUF VITAMIN-E-BASIS
ADMINISTRATION DE PREPARATIONS LIPOSOMALES DE COMPOSES A BASE DE VITAMINE E

(30) Priority: 19.12.2001 US 342156 P; 29.08.2002 US 406807 P; 15.10.2002 US 418602 P
(43) Date of publication of application: 06.10.2004
(73) Proprietor: RESEARCH DEVELOPMENT FOUNDATION, Carson City, Nevada 89703 (US)
(72) Inventor: SANDERS, Bob, G., Austin, TX 78720 (US); KLINE, Kimberly, Austin, TX 78703 (US); LAWSON, Karla, A., Austin, TX 78705 (US); MENCHACA, Marla, S., Austin, TX 78726 (US); KNIGHT, J., Vernon, Houston, TX 77027 (US); WELLEN, Clyde, W., Houston, TX 77019 (US)
(74) Representative: McQueen, Andrew Peter
(86) International application number: PCT/US2002/040846
(87) International publication number: WO 2003/053407

(56) References cited:
- EP-A1- 0 393 575
- WO-A-00/16772
- WO-A-00/71163
- WO-A-01/22937
- WO-A-01/26606
- US-A- 4 181 725
- US-A- 4 797 285
- US-A- 4 873 088
- US-A- 4 895 719
- US-A- 5 114 957
- US-A- 5 403 834
- US-A- 5 605 703
- US-A- 5 997 899

## Description

### Field of the Invention

This invention relates to the fields of pharmacology and cancer treatment. More specifically, this invention relates to delivery of liposomal preparations of vitamin E based compounds for the treatment and the prevention of cancer according to the claims.

### Description of the Related Art

Regulatory controls of pro-life (survival) and pro-death (apoptosis) are extremely complex, involving multiple intracellular signaling pathways and multiple interacting gene products. Cancer cells may exhibit enhanced expression of genes and their products that promote cell proliferation, allowing cancer cells to increase in number. In addition to enhanced expression of pro-life genes, cancer cells down-regulate genes and their products that control pro-death signals, resulting in the accumulation and enhanced metastasis of life threatening cancer cells. Combinations of unregulated cell proliferation and suppression of death inducing signaling pathways give cancer cells both growth and survival advantages.

Whether a cell increases in numbers or not depends on a balance of expression of negatively-acting and positively-acting growth regulatory gene products, and the presence or absence of functional cell death signaling pathways. Negative-acting growth regulatory genes contribute to blockage of cells in the cell cycle. Positive-acting growth regulatory genes stimulate cells to progress through the cell cycle. Genes involved in apoptosis can be either proapoptotic or antiapoptotic, and the dynamic balance between them determines whether a cell lives or dies.

A wide variety of pathological cell proliferative conditions exist for which novel therapeutic strategies and agents are needed to provide benefits. These pathological conditions may occur in almost all cell types capable of abnormal cell proliferation or abnormal responsiveness to cell death signals. Among the cell types that exhibit pathological or abnormal growth and death characteristics are fibroblasts, vascular endothelial cells, and epithelial cells. Thus, novel methods are needed to treat local or disseminated pathological conditions in all or almost all organ and tissue systems of individuals.

Most cancers, whether they be male specific such as prostate or testicular, or female specific such as breast, ovarian or cervical or whether they affect males and females equally such as liver, skin or lung, with time undergo increased genetic lesions and epigenetic events, and eventually may become highly metastatic and difficult to treat. Surgical removal of localized cancers has proven effective only when the cancer has not spread beyond the primary lesion. Once the cancer has spread to other tissues and organs, the surgical procedures must be supplemented with other more specific procedures to eradicate the diseased or malignant cells. Most of the commonly utilized supplementary procedures for treating diseased or malignant cells such as chemotherapy or radiation are not localized to the tumor cells and, although they have a proportionally greater destructive effect on malignant cells, often affect normal cells to some extent.

Some natural vitamin E compounds, and some derivatives of vitamin E have been used as proapoptotic and DNA synthesis inhibiting agents and thereby potent anti-cancer agents. Structurally, vitamin E is composed of a chromanol head and an alkyl side chain. There are eight major naturally occurring forms of vitamin E: alpha (α), beta (β), gamma (γ), and delta (δ) tocopherols and α, β, γ, and δ tocotrienols. Tocopherols differ from tocotrienols in that they have a saturated phytyl side chain rather than an unsaturated isoprenyl side chain. The four forms of tocopherols and tocotrienols differ in the number of methyl groups on the chromanol head (α has three, β and γ have two and δ has one) as shown in Table 1.

**TABLE 1**

| General structure of tocopherols and tocotrienols | | | |
|---|---|---|---|
| | **R¹** | **R²** | **R³** |
| Alpha (α) | CH₃ | CH₃ | CH₃ |
| Beta (β) | CH₃ | H | CH₃ |
| Gamma (γ) | H | CH₃ | CH₃ |
| Delta (δ) | H | H | CH₃ |

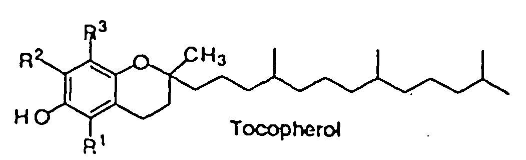

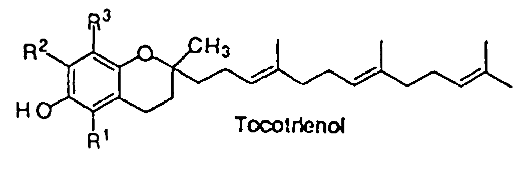

Several studies have described potent anti-tumor activity of RRR-α-tocopheryl succinate (vitamin E succinate; VES), a hydrolyzable ester derivative of RRR-α-tocopherol. Prasad and Edwards-Prasad were the first to describe the capacity of vitamin E succinate but not other forms of vitamin E to induce morphological alterations and growth inhibition of mouse melanoma B-16 cells and to suggest that vitamin E succinate might be a useful tumor therapeutic agent (1). Additional studies have demonstrated that vitamin E succinate is a potent growth inhibitor of a wide variety of epithelial cancer cell types, including breast, prostate, lung, and colon; as well as, hematopoietic-lymphoid leukemia and lymphoma cells, *in vitro* (2-7).

Recent studies have demonstrated vitamin E succinate to have anti-tumor activity in animal xenograft and allograft models when administered intraperitoneally (i.p.)(8-11), suggesting a possible therapeutic potential. Vitamin E succinate administered i.p. or orally (p.o.) has also been shown to have inhibitory effects on carcinogen [benzo(a)pyrene]-induced forestomach carcinogenesis in mice, suggesting potential as an anti-carcinogenic agent (12). Investigations have demonstrated that vitamin E succinate-induces concentration- and time-dependent inhibition of cancer cell growth via DNA synthesis blockage, induction of cellular differentiation, and induction of apoptosis (5, 6, 10, 13-15, unpublished data).

Inhibition of cell proliferation involves a GO/G1 cell cycle blockage, mediated, in part, by MAP kinases MEK1 and ERK1, and upregulation of the key cell cycle regulatory protein p21 (waf1/cip1) (30). Induction of differentiation is characterized by morphological changes, elevated beta casein message, expression of milk lipids, elevated cytokeratin 18 protein and down-regulation of Her2/neu protein (13). Differentiation is mediated, in part, by activation of MEK1, ERK1/2, and phosphorylation of the c-Jun protein (13, 14). Of the multiple apoptotic signaling events modulated by RRR-α-tocopherol succinate, especially noteworthy is its ability to convert Fas/Fas ligand non-responsive tumor cells to Fas/Fas ligand responsiveness and its ability to convert transforming growth factor-beta (TGF-α) non-responsive tumor cells to TGF-α responsiveness, with both restored pathways converging on JNK/c-Jun, followed by translocation of Bax protein to the mitochondria, induction of mitochondria permeability transition, followed by cytochrome c release to the cytoplasm, activation of caspases 9 and 3, cleavage of poly (ADP-ribose) polymerase (PARP), and apoptosis (15, 29, 31).

Vitamin E succinate is noteworthy not only for its induction of growth inhibitory effects on tumor cells but also for its lack of toxicity toward normal cells and tissues (2-7, 11). The use of a non-hydrolyzable vitamin E succinate derivative has shown that it is the intact compound and not either of its cleavage products (namely, RRR-α-tocopherol or succinic acid), that are responsible for the anti-proliferative effects (4). Thus, the anti-proliferative actions of this vitamin E derivative are considered to be due to non-antioxidant properties.

RRR-α-tocopheryl succinate (VES) is a derivative of RRR-α-tocopherol that has been structurally modified via an ester linkage to contain a succinyl moiety instead of a hydroxyl moiety at the 6-position of the chroman head. This ester linked succinate moiety of RRR-α-tocopherol has been the most potent form of vitamin E affecting the biological actions of triggering apoptosis and inhibiting DNA synthesis. This form of vitamin E induces tumor cells to undergo apoptosis, while having no apoptotic inducing effects on normal cells. The succinated form of vitamin E is effective as an anticancer agent as an intact agent; however, cellular and tissue esterases that can cleave the succinate moiety, thereby converting the succinate form of RRR-α-tocopherol to the free RRR-α-tocopherol, render this compound ineffective as an anticancer agent. RRR-α-tocopherol exhibits neither antiproliferative nor proapoptotic biological activity in cells of epithelial or immune origin.

Construction of RRR-alpha-tocopherol or RRR-alpha-tocotrienol based compounds modified at the C6 position of the first ring of the chromonal head of alpha-tocopherol or alpha-tocotrienol via an ether linkage would provide compounds with potent anticancer properties. Cellular etherases have not been reported in cells; thus, such compounds will remain intact in cell culture as well as *in vivo.* In US 6,417,223 commercially available RRR-α-tocopherol in pure form was used as the starting material from which to synthesize vitamin E analogs. Modifications were made to three parts of the RRR-α-tocopherol molecule: to the number 6 carbon of the phenolic ring in the chroman head, to the chroman head, comprising the phenolic and heterocyclic rings, or to the phytyl tail. In RRR-α-tocopherol the number 6 carbon of the phenolic ring has a hydroxyl (-OH) moiety which is critical for antioxidant activity.

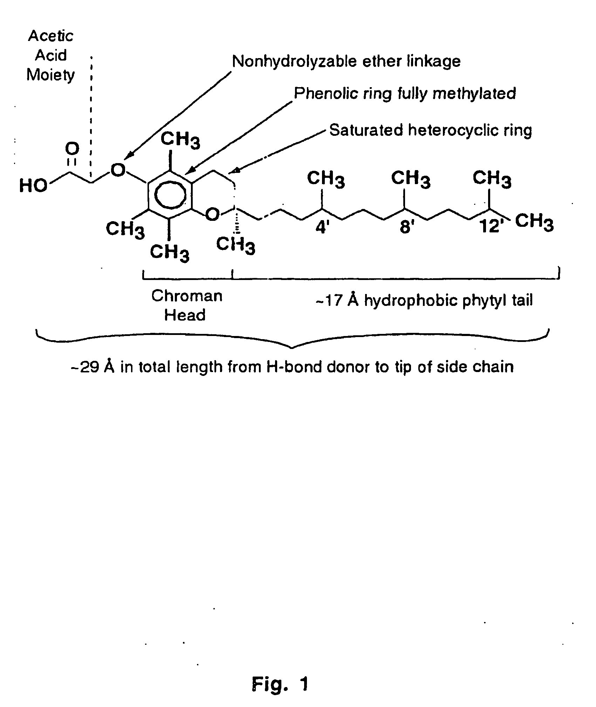

Screening of the analogs for ability to induce apoptosis in a wide variety of human cancer cells, but not normal human cells, together with structure function analyses of the analogs suggest that tocopherol-based analogs of 29 Å in total length from H-bond donor to tip of phytyl tail, which itself is 17 Å in length, having a fully methylated closed phenolic ring, saturated closed heterocyclic ring, and a nonhydrolyzable acetic acid moiety attached to C6 of the phenolic ring with an ether linkage exhibit the most potent anti-cancer activity (Fig. 1).

The method of delivery of therapeutic agents, whether it is by oral, dietary, gavage, subcutaneous, intraperitoneal, topical, intravenous, intramuscular, respiratory, etc., has a major influence on levels and tissue distribution of drugs. U.S. Patent No. 6,090,407 demonstrates that anti-cancer drugs paclitaxel and camptothecin can be incorporated into liposomes for delivery to the respiratory tract of an individual via nebulization. The administration of these anti-cancer drugs by liposomal inhalation is a faster and more efficient means of delivery than either intramuscular injection or oral administration.

The use of a liposomal aerosol for delivery of the plant alkaloid 9-nitrocamptothecin is a superior method for inhibition of human breast (28), colon, and lung cancer xenografts in immune compromised nude mice when compared to delivery of 9 - nitrocamptothecin by intramuscular injection. Levels of 9 - nitrocamptothecin, after thirty minutes by aerosol liposome method of delivery, in the lungs, liver and brain were 310 ng/g, 192 ng/g, and 61 ng/g, respectively; whereas, levels of 9-nitrocamptothecin, after thirty minutes by intramuscular delivery, in the lungs, liver, and brain were 2-4 ng/g, 136 ng/g, and 0, respectively (16). In addition, this method of delivery appears to be highly effective against pulmonary metastasis of melanoma and osteosarcoma in mice (18). Of major importance, aerosol delivery of drugs shows increased efficacy and is well tolerated by humans (19). Thus, the aerosol liposome method for delivery of drugs is effective in achieving higher levels and greater tissue distribution.

Additionally, the inventors have recognized a need for further effective methods of liposomal delivery of vitamin E based anticancer drugs that provide for longer retention, higher drug concentration, reduced systemic toxicity and reduced dosage requirements. Thus, the prior art is deficient in the lack of an effective means of delivering vitamin E based anticancer drugs via liposomesal to an individual. Specifically, the aerosol liposomal delivery or administration by gavage of liposomal compositions of vitamin E based anticancer drugs is desired. The present invention fulfills this long-standing need and desire in the art.

### SUMMARY OF THE INVENTION

The invention provides a vitamin E based anti-cancer compound selected from the group consisting of
a) a compound having the structural formula 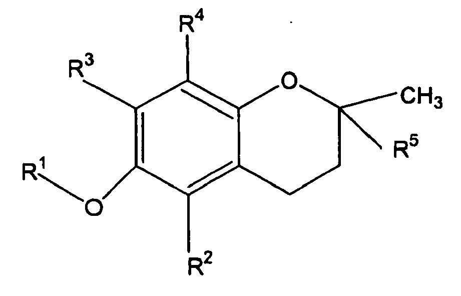 wherein R¹ is a carboxylic acid;
   R² and R³ are hydrogen or R⁴;
   R⁴ is methyl; and
   R⁵ is alkyl;
b) α-tocotrienol;
c) β-tocotrienols;
d) γ-tocotrienol; and
e) δ-tocotrienol,
for use in the treatment of a cell proliferative disease, wherein said compound is to be administered by aerosol delivery of a composition comprising a liposome comprising said compound and a lipid.

The invention further provides the use of a vitamin E based anti-cancer compound selected from the group of a) to e) recited above in the preparation of a medicament for the treatment of a cell proliferative disease in an individual, wherein said compound is to be administered by aerosol delivery of a composition comprising a liposome comprising said compound and a lipid.

Other and further aspects, features, benefits, and advantages of the present invention will be apparent from the following description of the presently preferred embodiments of the invention given for the purpose of disclosure.

### BRIEF DESCRIPTION OF THE DRAWINGS

So that the matter in which the above-recited features, advantages and objects of the invention, as well as others which will become clear, are attained and can be understood in detail, more particular descriptions of the invention are briefly summarized above may be had by reference to certain embodiments thereof which are illustrated in the appended drawings. These drawings form a part of the specification. It is to be noted; however, that the appended drawings illustrate preferred embodiments of the invention and therefore are not to be considered limiting in their scope.

**Figure 1** is a model of R,R,R-α-tocopherol based analogs depicting the structural elements required for potent anti-cancer activity. The R¹ group side chain at C6 must be of a length such that the total length of the molecule does not exceed 29Å.

**Figure 2** compares the anticancer effects of natural alpha- and gamma-tocopherols, natural alpha-tocotrienol, tocotrienol enriched fraction (TRF), synthetic tocopherols and synthetic tocopherol derivatives on 66 c1.4 GFP mammary tumor cells.

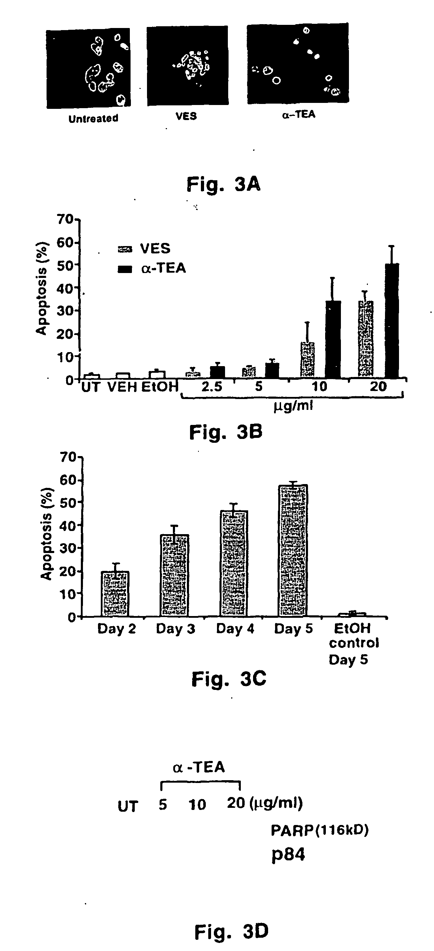

**Figures 3A-3D** depict α-TEA induced apoptosis. **Figure 3A**: 66 cl.4-GFP murine mammary cells were treated with 10 µg/ml of α-TEA or vitamin E succinate (positive control) or untreated and cultured for 3 days. Cells were harvested, nuclei were labeled with the fluorescent DNA-binding dye DAPI and cells were examined using a Zeiss ICM 405 fluorescent microscope (x400), using a 487701 filter. Nuclei of cells with condensed chromatin or fragmented nuclei were scored as apoptotic. Data are representative of numerous experiments. **Figures 3B/3C****:** Analyses of nuclei of DAPI stained cells show α-TEA to induce apoptosis in a concentration- and time- dependent manner. Data are depicted as mean ± S.D. of three experiments. **Figure 3D****:** Additional evidence of α-TEA induction of apoptosis by poly (ADP-ribose) polymerase (PARP) cleavage. 66 cl.4-GFP cells were treated with 5, 10, or 20 µg/ml α-TEA for 48 hours, cellular lysates were analyzed for PARP cleavage product (p84) by western immunoblot analyses. Data are representative of 3 separate experiments.

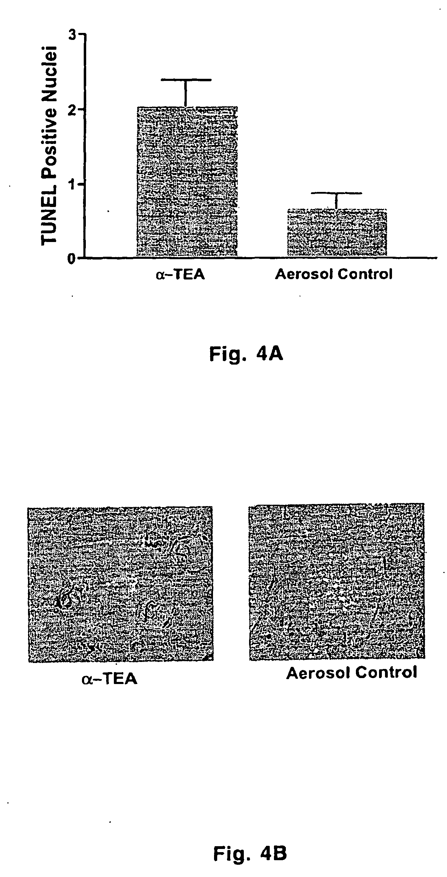

**Figures 4A** and **4**B depict that α-TEA induces 66 cl.4 cells to undergo apoptosis *in vivo*. α-TEA induction of apoptosis was determined using 5 µm tumor sections derived from liposomal α-TEA/aerosol treatment and liposome aerosol control group animals (N = 4). Apoptotic cells were determined using ApopTag *In Situ* Apoptosis Detection kit (Intergen, Purchase, NY). **Figure 4A** compares the number of apoptotic nuclei in liposomal α-TEA aerosol and aerosol treated controls. **Figure 4B** depicts positive stained apoptotic cells in tumor sections from liposomal α-TEA aerosol and control treated mice.

**Figure 5** depicts α-TEA inhibition of 66 c1.4-GFP clonal growth. Treatment of 66 c1.4-GFP cells (plated at 600 cells/tissue culture plate) with α-TEA at 1.25, 2.5, and 5 µg/ml for 10 days inhibited colony formation. Cells were stained with methylene blue and the number of colonies in treatment and control groups were counted.

**Figure 6** depicts body weight of balb/c mice implanted with 66 cl.4 GFP murine mammary tumor cells and treated with liposome/α-TEA composition delivered via aerosol. Animal weight is monitored from day nine after implantation.

**Figure 7** depicts serum and tissue levels of α-TEA in balb/c mice 0, 2, 6, or 24 hours after liposomal/α-TEA 1 aerosol treatment ended.

**Figure 8** depicts tumor weights in balb/c mice implanted with 66 c1.4 GFP murine mammary tumor cells and treated with liposomal/α-TEA delivered via aerosol. Tumor weight is monitored from day nine after implantation.

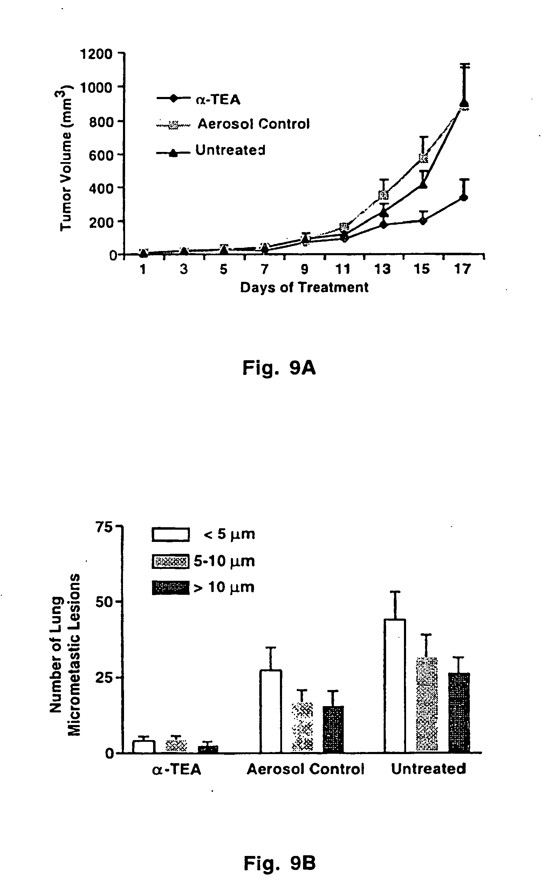

**Figures 9A** and **9B** depict the inhibition of tumor growth and micrometatases with liposomal aerosol α-TEA. **Figure 9A**: 66 cl.4-GFP cells at 2 x 10⁵/mouse were injected into the inguinal area at a point equal distance between the 4th and 5 th nipples. Nine days after tumor inoculation, mice (10/group) were not treated or treated daily with liposomal α-TEA/aerosol or aerosol only for 17 days. Tumor volume/mouse was determined at two-day intervals. Tumor volume (mm³) are depicted as mean ± S. E. **Figure 9B**: At necropsy, the number of fluorescent micrometastases in the left lung lobe from liposomal α-TEA/aerosol (8 mice), aerosol only (10 mice), and untreated mice (10 mice) were determined using a Nikon fluorescent microscope (TE-200; 200X), and Image Pro-Plus software was used for determining size of micrometastases. Data are depicted as mean ± S. E.

**Figure 10** depicts the inhibition of tumor growth of 66 cl.4 GFP mammary tumor cells by liposomal α-TEA and liposomal vitamin E succinate (VES) delivered via aerosol nebulization.

**Figure 11** depicts tumor weights in balb/c mice implanted with 66 cl.4 GFP murine mammary tumor cells and treated with liposomal/ α-TEA delivered via gavage. Tumor weight is monitored from day nine after implantation. Figure 1 1 differs from Figure 8 only in that the mice were treated daily by gavage with 5 mg α-TEA in peanut oil or received peanut oil only, and were treated for only 13 days.

**Figures 12A** and **12B** depict that α-TEA administered by gavage does not inhibit tumor growth at the site of inoculation but does inhibit lung micrometastases. Tumor volume and micrometastases data determinations were as described in the legend to Figures 9A and 9B.

**Figures 13** depicts the inhibition of tumor growth of 66 cl.4 GFP mammary tumor cells by liposomal α-TEA and liposomal vitamin E succinate (VES) delivered via gavage.

**Figures 14A** and **14B** depict the inhibition of lung metastases (Figure 14A) and lymphnode metastases (Figure 14B) of 66 c1.4 GFP mammary tumor cells by liposomal α-TEA and liposomal vitamin E succinate (VES) delivered via gavage.

**Figure 15** depicts the inhibition of tumor growth of MDA-MB-435 breast cancer cells by liposomal α-TEA delivered via gavage.

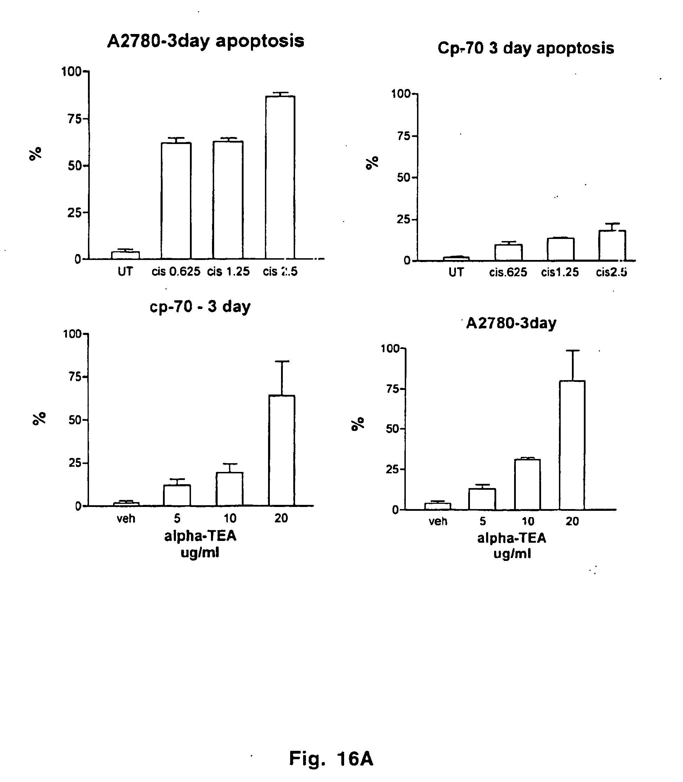

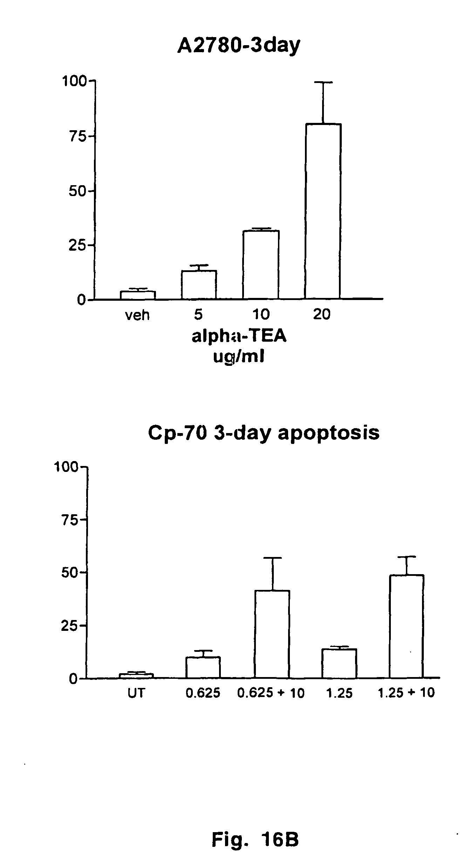

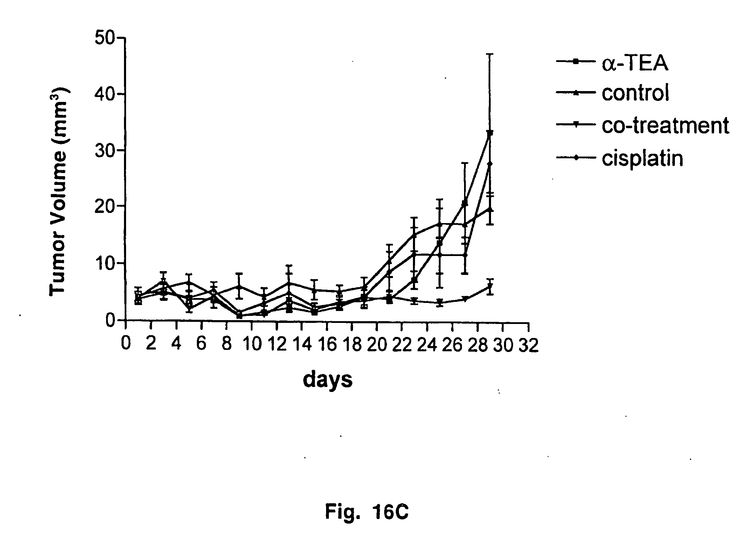

**Figures 16A-16C** depict the apoptotic effects on of α-TEA or α-TEA/cisplatin in combination on the A2780 **cisplatin-**sensitive cell line and on cisplatin-insensitive cp-70 human ovarian cancer cells. **Figure 16A**: Apoptotic effect *in vitro* of α-TEA on the A2780 and cp-70 human ovarian cancer cell lines. **Figure 16B**: α-TEA restores cisplatin sensitivity to cp-70 *in vitro.* **Figure 16C**: the growth inhibitory effect of both α-TEA and cisplatin in combination on A2780 and cp-70 *in vivo.* In vivo data show that α-TEA converts cp-70 cisplatin cells to cisplatin sensitivity, and that combinations of α-TEA + cisplatin reduce the growth of cp-70 ovarian cell xenografts in nude mice.

**Figure 17** depicts the effect of α-TEA, methylselenocysteine and trans-resveratrol on MDA-MB-435 GFP FL breast cancer tumor growth *in vivo.*

### DETAILED DESCRIPTION OF THE INVENTION

In one embodiment of the present invention, there is provided a vitamin E based anti-cancer compound selected from the group consisting of
a) a compound having the structural formula 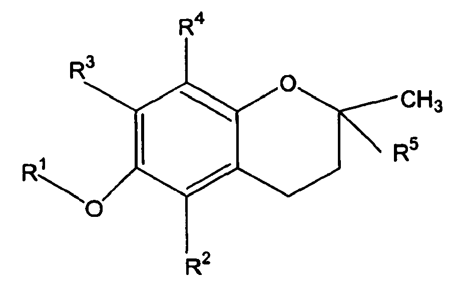 wherein R¹ is a carboxylic acid;
   R² and R³ are hydrogen or R⁴;
   R⁴ is methyl; and
   R⁵ is alkyl;
b) α-tocotrienol;
c) β-tocotrienol;
d) γ-tocotrienol; and
e) δ-tocotrienol,
for use in the treatment of a cell proliferative disease, wherein said compound is to be administered by aerosol delivery of a composition comprising a liposome comprising said compound and a lipid.

The invention further provides the use of a vitamin E based anti-cancer compound selected from the group of a) to e) recited above in the preparation of a medicament for the treatment of a cell proliferative disease in an individual, wherein said compound is to be administered by aerosol delivery of a composition comprising a liposome comprising said compound and a lipid.

In all aspects of this embodiment the vitamin E based anticancer compounds may be a tocopherol such as β- tocopherol, γ-tocopherol, 8-tocopherol, or 2,5,7,8-tetramethyl-(2R-(4R,8R,12-trimethyltridecyl) chroman-6-yloxy) acetic acid. Additionally, the vitamin E based anticancer compounds may be tocotrienol such as α- tocotrienol, β- tocotrienol, γ- tocotrienol, δ- tocotrienol, or tocotrienol enriched fraction, or a synthetic vitamin E based compound such as d1-α-tocopherol, d1-α-tocopherol acetate, d1-α-tocopherol nicotinate, or d1-α-tocopherol phosphate.

A representative example of a suitable lipid in the liposome is 1,2-dilauroyl-sn-glycero-3-phosphocholine. A preferred example is a liposome with a final concentration of the vitamin E based anti-cancer compound in the liposome that is no greater than 20.0 mg/ml. The liposome comprising said vitamin E based compound may be delivered, for example, via aerosol nebulization or an aerosol inhaler. A preferred means of delivery is a liposomal aerosol via a jet nebulizer.

In an aspect to this embodiment the treatment may further comprise administration of a second composition of an anticancer drug contained within a delivery vehicle. The second composition may be administered in combination with or sequentially to the vitamin E based compound liposome composition. When administration of the compositions is combined, the vitamin E based compound and the anticancer drug may be contained within the same liposome. Representative examples of anticancer drugs are anticancer drug is 9-nitrocamptothecin, cisplaten, paclitaxel, doxirubicin, or celecoxib.

The vitamin B based anti-cancer compounds of the instant invention exhibit an anti-proliferative effect comprising apoptosis, DNA synthesis arrest, cell cycle arrest, or cellular differentiation. In this embodiment the quantitative and/or qualitative analysis of the antiproliferative effect may be determined by detecting a biomarker. A preferred example of a biomarker is the cell proliferation marker KI-67. Alternatively, a immunohistochemical assay may be used.

Delivery of the vitamin B based or other anti-cancer compounds of the present invention may be used to treat neoplastic diseases and non-neoplastic diseases. Representative examples of neoplastic diseases are ovarian cancer, cervical cancer, endometrial cancer, bladder cancer, lung cancer, cervical cancer, breast cancer, prostate cancer, testicular cancer, gliomas, fibrosarcomas, retinoblastomas, melanomas, soft tissue sarcomas, osteosarcomas, colon cancer, carcinoma of the kidney, pancreatic cancer, basal cell carcinoma, and squamous cell carcinoma. Representative examples of non-neoplastic diseases are selected from the group consisting of psoriasis, benign proliferative skin diseases, ichthyosis, papilloma, restenosis, scleroderma and hemangioma, and leukoplakia.

Vitamin E based anti-cancer compounds of the present invention may be used to treat non-neoplastic diseases that develop due to failure of selected cells to undergo normal programmed cell death or apoptosis. Representative examples of diseases and disorders that occur due to the failure of cells to die are autoimmune diseases. Autoimmune diseases are characterized by immune cell destruction of self cells, tissues and organs. A representative group of autoimmune diseases includes autoimmune thyroiditis, multiple sclerosis, myasthenia gravis, systemic lupus erythematosus, dermatitis herpetiformis, celiac disease, and rheumatoid arthritis. This invention is not limited to autoimmunity, but includes all disorders having an immune component, such as the inflammatory process involved in cardiovascular plaque formation, or ultra violet radiation induced skin damage.

Vitamin E based anti-cancer compounds of the present invention may be used to treat disorders and diseases that develop due to viral infections. Representative examples of diseases and disorders that occur due to viral infections are human immunodeficiency viruses (HIV). Since vitamin E based compounds work on intracellular apoptotic signaling networks, a delivery vesicle, such as a liposomal aerosol, containing the vitamin E based anti-cancer compounds of the present invention have the capacity to impact signal transduction of any type of external cellular signal such as cytokines, viruses, bacteria, toxins, heavy metals, etc.

Vitamin E based anti-cancer compounds of the present invention are delivered in a liposome. The liposome may further comprise an anticancer drug. In a preferred aspect, the liposome has a ratio of vitamin E based anticancer compound to lipid of about 1:3 wt:wt. The vitamin E based anti-cancer compounds, the anticancer drugs and the delivery methods may be those as disclosed *supra.*

The following definitions are given for the purpose of facilitating understanding of the inventions disclosed herein. Any terms not specifically defined should be interpreted according to the common meaning of the term in the art.

As used herein, the terms "aerosol" "gavage", "liposome" "delivery vesicle" and "vesicle" shall include different chemical compositions for vesicle/liposome preparations and different methodologies for aerosol dispersal or oral delivery of these preparations.

As used herein, the term "individual" shall refer to animals and humans.

As used herein, the term "biologically inhibiting" or "inhibition" of the growth of syngenic tumor grafts shall include partial or total growth inhibition and also is meant to include decreases in the rate of proliferation or growth of the tumor cells. The biologically inhibitory dose of the composition of the present invention may be determined by assessing the effects of the test element on target malignant or abnormally proliferating cell growth in tissue culture, tumor growth in animals and cell culture or any other method known to those of ordinary skill in the art.

As used herein, the term "inhibition of metastases" shall include partial or total inhibition of tumor cell migration from the primary site to other organs, specifically the lungs in the data provided above. The biological metastatic inhibitory dose of the composition of the present invention may be determined by assessing the effects of the test element on target malignant or abnormally proliferating cell growth in tissue culture, tumor growth in animals and cell culture or any other method known to those of ordinary skill in the art.

As used herein, the term "inhibition of angiogenesis" shall include partial or total inhibition of tumor blood vessel formation or reduction in blood carrying capacity of blood vessels supplying blood to tumors.

As used herein, the term "induction of programmed cell death or apoptosis" shall include partial or total cell death with cells exhibiting established morphological and biochemical apoptotic characteristics. The dose of the composition of the present invention that induces apoptosis may be determined by assessing the effects of the test element on target malignant or abnormally proliferating cell growth in tissue culture, tumor growth in animals and cell culture or any other method known to those of ordinary skill in the art.

As used herein, the term "induction of DNA synthesis arrest" shall include growth arrest due to treated cells being blocked in GO/G1, S, or G2/M cell cycle phases. The dose of the composition of the present invention that induces DNA synthesis arrest may be determined by assessing the effects of the test element on target malignant or abnormally proliferating cell growth in tissue culture, tumor growth in animals and cell culture or any other method known to those of ordinary skill in the art.

As used herein, the term "induction of cellular differentiation" shall include growth arrest due to treated cells being induced to undergo cellular differentiation as defined by established morphological and biochemical differentiation characterization, a stage in which cellular proliferation does not occur. The dose of the composition of the present invention that induces cellular differentiation may be determined by assessing the effects of the test element on target malignant or abnormally proliferating cell growth in tissue culture, tumor growth in animals and cell culture or any other method known to those of ordinary skill in the art.

As used herein, "α-TEA" shall include an RRR-α-tocopherol ether-linked acetic acid analog which is a non-hydrolyzable ether analog of RRR-α-tocopherol, i.e., 2,5,7,8-tetramethyl-2R-(4R,8R-12- trimethyltridecyl)chroman-6-yloxy acetic acid which can also be abbreviated as RRR-α-tocopheryloxyacetic acid.

Provided herein are natural or synthetic vitamin E based anti-cancer drugs for used in treating cell proliferative diseases via aerosol delivery of liposomal compositions comprising said drugs and a lipid. The vitamin E based compounds of the instant invention exhibit an anti-proliferative effect; representative examples of these anti-proliferative effects are apoptosis, DNA synthesis arrest, cell cycle arrest, or cellular differentiation. These compounds exhibit potent anti-metastatic effects and do not exhibit toxicity to normal cells and tissue *in vivo* when administered by a clinically relevant route such as aerosol delivery or gavage.

Optionally, the vitamin E based anticancer drug and a second anti-cancer drug, such as, but not limited to, 9-nitrocamptothecin (9NC), doxorubicin, paxitaxol, celecoxib, or cisplatin, in a liposomal composition or administered via another route, such as intraperitoneal injection, can be administered in combination or sequentially with α-TEA. For example, 9-nitrocamptothecin, as a topoisomerase-1 inhibitor which causes DNA single-strand breaks that are converted into DNA double strand breaks during DNA replication, has a different cell killing mechanism from α-TEA. Furthermore, 9NC can activate a traditional CD95/CD95Ligand (FADD/caspase 8) dependent apoptotic pathway (29) which might synergize with the nontraditional pathway, i.e., CD95/Daxx/JNK/Mitochondria. This provides for enhanced cell killing.

These vitamin E based compounds of the instant invention include natural or synthetic tocopherols or tocotrienols and derivatives thereof having chemical funtionalization at position R¹ of the chroman structure and position R⁵ of the phytyl or isoprenyl side chains. R¹ is a carboxylic acid, e.g. acetic acid. Generally, synthesis of these compounds is accomplished by reacting R,R,R-alpha-tocopherol with the appropriate bromoalkanoic acid using methods standard in the art.

Derivatives of the natural tocopherols and tocotrienols are non-hydrolizable by cellular esterases and, although not limited to such a C6 side chain, preferably have an acetic acid moiety in this position. A preferred compound that fulfills the structural elements required for potent anti-cancer activity is α-TEA which differs from RRR-α-tocopherol by an acetic acid moiety linked to the phenolic oxygen at carbon 6 of the chroman head by an ether linkage. VES differs from α-TEA in that a succinic acid moiety is linked by an ester linkage to the phenol at carbon 6 of the chroman head. Since the antioxidant properties of the parent compound, RRR-α-tocopherol, reside in the -OH moiety at carbon 6, the anti-tumor properties of α-TEA are not antioxidant mediated.

The present invention may be used as a therapeutic agent. The methods of the present invention may be used to treat any animal. Most preferably, the methods of the present invention are useful in humans. Generally, to achieve pharmacologically efficacious cell killing and anti-proliferative effects, the liposomal/anti-cancer compound compositions may be administered in any therapeutically effective dose. The dosage administered is dependent upon the age, clinical stage and extent of the disease or genetic predisposition of the individual, location, weight, kind of concurrent treatment, if any, and nature of the pathological or malignant condition. Preferably, the dosage is from about 0.1 mg/kg to about 100 mg/kg. A person having ordinary skill in this art would readily be able to determine, without undue experimentation, the appropriate dosages.

*In vivo* studies of tumor growth and metastasis of human tumor cells either ectopically or orthotopically transplanted into immune compromised animals, such as nude mice, or *in vivo* studies employing well recognized animal models provides pre-clinical data for clinical trials. Without being limited to these animal models, such *in vivo* studies can focus on other neoplastic and non-neoplastic models of cell proliferative diseases. For example, the metastatic non-estrogen responsive MDA-MB-435 breast cancer cells, 66 cl.4 GFP cells or cisplatin resistant cp-70 human ovarian cancer cells can be used.

The use of additional novel tocopherol, tocotrienol, and other chroman derivatives with or without derivatives of saturated phytyl or unsaturated isoprenyl side chains or analogs thereof in a liposomal composition for delivery to an individual such as aerosol delivery to the respiratory tract or delivery via gavage, is specifically contemplated. These molecules include chemical funtionalization of positions R¹-R⁵ of the chroman structure, and chemical functionalization of the phytyl and isoprenyl side chains, particularly compounds based on tocopherols and tocotrienols. Additionally, compounds with heteroatom substitutions (N or S) for the chroman ring oxygen and the oxygen of the 6-hydroxy group are contemplated.

Using alkylation chemistry, a large number of compounds containing different R' groups can be synthesized. After alkylation, further chemical modification of the R' groups permits the synthesis of a wide range of novel compounds. In the vitamin E based compounds of the invention, the R¹ substituent is a carboxylic acid.

Bromination of the benzylic methyl groups of the chroman group provide intermediates that permit variation of the R², R³ and R⁴ groups. Substituents for R² and R³ may be hydrogen or R⁴. R⁴ is methyl. R⁵ is alkyl.

It is also contemplated that other lipids may be used in the liposomal composition. Any lipid that can incorporate these vitamin E based anti-cancer compounds or other anticancer compounds and deliver a therapeutic dosage would be suitable. Liposomal delivery is via aerosolization. For example, methods of aerosolization and nebulization for liposomal delivery can be used.

The following examples are given for the purpose of illustrating various embodiments of the invention and are not meant to limit the present invention in any fashion.

### EXAMPLE 1

### Synthesis and characterization of 2,5,7,8-tetramethyl-(2R-(4R,8R,12-trimethyltridecyl) chroman-6-yloxy) acetic acid (α-TEA)

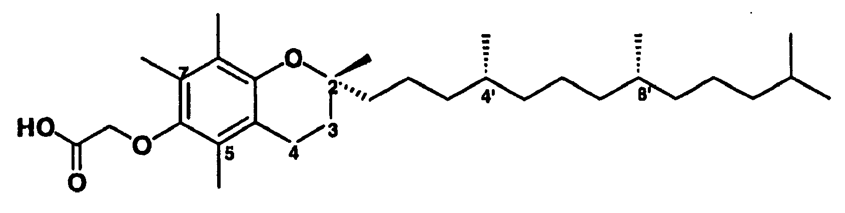

For scaled-up production, α-TEA was prepared as follows. NaH (5.0 g, 124.9 mmol) was suspended in dry THF (300 ml) and stirred under argon at 0 °C for 10 min. prior to the addition via cabbula of α-tocopherol (41.3 g, 96.1 mmol) that was dissolved in 100ml of dry THF. This mixture was stirred at 0 °C for 15 min while under argon pressure, then ethyl bromoacetate (19.26 g, 115.3 mmol) was added via syringe. The reaction was monitored by TLC (hexane : ethyl acetate = 10 : 1, R_{f}= 0.65) and was completed in 3.5 hours. The reaction mixture was diluted with 150 ml of CH₂Cl₂, washed with saturated NaCl solution (150 ml x 3) until the organic phase was clear, dried over anhydrous Na₂SO₄, and the solvent removed under a reduced pressure. The crude product still contained a small amount of free α-tocopehrol which could be removed by column chromatography on silica gel using hexane: ethyl acetate (30:1 to 20:1) to yield pure product α-TEA ethyl ester (41.6 g, 84%).

The α-TEA ethyl ester (21.0 g, 40.7 mmol) was dissolved in 250 ml of THF, then 75 ml of 10% KOH (122.1 mmol) was added and the mixture stirred at room temperature for 6 hours. The reaction was monitored by TLC (CHCl₃ : MeOH : CH₃COOH = 97 : 2.5 : 0.5, R_{f} = 0.18) and was quenched with 100 ml of water. The solution was adjusted to pH 3 using 1N HCl and the product extracted with CH₂Cl₂ (100 ml x 4), washed with saturated NaCl solution, dried over Na₂SO₄ and the solvent was removed under a reduced pressure, providing the final product α-TEA as a white waxy solid (18.5 g, 93%). Melting point: 54-55°C; Molecular weight: 488.8

### EXAMPLE 2

### Murine Mammary Tumor Cell Line

66 cl.4-GFP cells are a mouse mammary tumor cell line originally derived from a spontaneous mammary tumor in a Balb/cfC3H mouse and later isolated as a 6-thioguanine-resistant clone (20, 21). Subsequently these cells were stably transfected with the enhanced green fluorescent protein (GFP). 66 cl.4-GFP cells are highly metastatic with 100% micrometastases to the lungs. Prior to use in these studies, cells were sent to the University of Missouri Research Animal Diagnostic and Investigative Laboratory (RADIL; Columbia, MO) where they were certified to be pathogen free.

66 cl.4 GFP cells were maintained as monolayer cultures in growth media: McCoy's media (Invitrogen Life Taechnologies, Carlsbad, CA.) supplemented with 10% fetal bovine serum (FBS, Hyclone Lab, Logan, UT), 100 µg/ml streptomycin, 100 IU/ml penicillin, 1 X (vol/vol) non-essential amino acids, 1X (vol/vol) MEM vitamins, 1.5 mM sodium pyruvate, and 50 µg/ml gentamycin (Sigma Chemical Co., St. Louis, MO). Treatments were given using this same McCoy's supplemented media expect FBS content was reduced to 5%. Cultures were routinely examined to verify absence of mycoplasma contamination.

### EXAMPLE 3

### Determination of apoptosis by morphological evaluation of DAPI-stained nuclei

Apoptosis was determined using previously published procedures (22). Briefly, 1x10⁵ cells/well in 12-well plates were cultured overnight to permit attachment. Next, the cells were treated with α-TEA, vitamin E succinate (Sigma) or ethanol control (0.1% ethanol F.C. vol/vol) in experimental media at various concentrations of α-TEA and vitamin E succinate for various time intervals. After treatment, floating cells plus scraped-released adherent cells were pelleted by centrifugation for 5 min at 350 X g, washed one time with phosphate buffered saline (PBS; 137 mM NaCl, 2.7 mM KCL, 10.4 mM Na₂HPO₄, 10.5 mM KH₂PO₄; pH 7.2), and stained with 2 µg/ml of 4',6-diamidino-2-phenylindole dihydrochloride (DAPI, Boehringer Mannheim, Indianapolis, IN) in 100% methanol for 15 min at 37°C.

Cells were viewed at 400X magnification with a Zeiss ICM 405 fluorescent microscope using a 487701 filter. Cells in which the nucleus contained condensed chromatin or cells exhibiting fragmented nuclei were scored as apoptotic. Data are reported as percentage of apoptotic cells per cell population, i.e., number apoptotic cells/total number of cells counted. Three different microscopic fields were examined and 200 cells counted at each location for a minimum of 600 cells counted per slide. Apoptotic data are presented as mean ± S.D. for three independently conducted experiments.

### EXAMPLE 4

### Western Immunoblot Detection of Poly (ADP-Ribose) Polymerase (PARP) Cleavage Fragment

Poly (ADP-Ribose) Polymerase cleavage was analyzed as an alternate method for detecting apoptosis. 66 cl.4-GFP cells were treated as described above for the DAPI assay. Following the PBS wash, cells were suspended in lysis buffer (1X PBS, 1% Nonidet P-40, 0.5% sodium deoxycholate, 0.1% sodium dodecyl sulfate (SDS), 1 µg/ml leupeptin, 1 µg/ml aprotinin, 1mM dithiothreitol (DTT), 2 mM sodium orthovanadate, 10 µg/ml phenylmethylsulfonyl fluoride (PMSF)) for 30 min at 4°C, vortexed, and supernatants collected by centrifugation at 15,000 X g for 20 min. Protein concentrations were determined using the Bio-Rad (Bradford) protein assay (Bio-Rad Laboratories, Hercules, CA), and samples (100 µg/lane) resolved on 7.5% SDS-polyacrylamide gels electrophoresed under reducing conditions.

Proteins were electroblotted onto a nitrocellulose membrane (0.2 µM pore Optitran BA-S-supported nitrocellulose; Schleicher and Schuell, Keene, NH). After transfer, membranes were blocked with blocking buffer [25 mM Tris-HCl (pH 8.0), 125 mM NaCl, 0.5% Tween-20, and 5% non-fat dry milk] for 45 min at room temperature. Immunoblotting was performed using 1 µg of primary rabbit anti-human Poly (ADP-Ribose) Polymerase antibody [PARP (H-250), Santa Cruz Biotechnology, Santa Cruz, CA], and horseradish peroxidase-conjugated goat anti-rabbit immunoglobulin was used as the secondary antibody (Jackson Immunoresearch Laboratory, West Grove, PA) at a 1:3,000 dilution. Horseradish peroxidase-labeled bands from washed blots were detected by enhanced chemiluminescence (Pierce, Rockford, IL) and autoradiography (Kodak BioMax film; Rochester, NY).

### EXAMPLE 5

### Colony-forming Assay

66 cl.4-GFP cells were seeded at 600 cells per 35 X 1 0 mm tissue culture plate (Nunclon, Rochester, NY) and allowed to adhere over night at 37° C. The next day, growth media were removed and replaced with treatment media containing α-TEA at 1.25, 2.5, 5, and 10 pg/ml, ethanol control (0.1% ethanol F.C. vol/vol), or media alone (untreated). Treatments were left on the cells for 10 days without change of media. After 10 days, media were removed and plates were washed with PBS three times. Cells were stained for 30 min with 1% methylene blue in PBS and colonies > 0.5 mm manually counted.

### EXAMPLE 6

### Balb/c Mice

Female Balb/cJ mice at 6 weeks of age (25 gm body weight) were purchased from Jackson Labs (Bar Harbor, ME), and were allowed to acclimate at least one week. Animals were housed at the Animal Resource Center at the University of Texas at Austin at 74 ± 2°F with 30-70% humidity and a 12 h alternating light-dark cycle. Animals were housed 5/cage and given water and standard lab chow *ad libitum.* Guidelines for the humane treatment of animals were followed as approved by the University of Texas Institutional Animal Care and Use Committee.

### EXAMPLE 7

### Tumor Cell Inoculation

66 cl.4-GFP cells were harvested by trypsinization, centrifuged, resuspended in McCoy's media, containing no supplements at a density of 2 X 10⁵/100µl. Mice were injected in the inguinal area at a point equal distance between the 4^{th} and 5^{th} nipples on the right side using a 23 gauge needle.

50 mice were assigned (10 per group) to aerosol treatment, aerosol control, oral treatment, oral control, or to the no treatment group so that the average tumor volume for all groups were closely matched. Each group had a range of 2 x 2-4 x 4 m m tumors at the start of treatments which were begun nine days following tumor cell inoculation. Ten additional mice, not injected with tumor cells, were treated with aerosol or oral α-TEA (5 each) for 17 days, removed from treatment and observed for an additional 11 months to evaluate long term safety. Tumors were measured using calipers every other day, and volumes were calculated using the formula: volume (mm³) = [width (mm)² X length (mm)] /2. Body weights were determined weekly.

### EXAMPLE 8

### Preparation and Administration of α-TEA Solubilized in Peanut Oil for Delivery by Gavage

α-TEA was dissolved in 100% ethanol (400 mg/ml) and then mixed with peanut oil (100% peanut oil; nSpired Natural Foods, San Leandro, CA) at a ratio of 1:8 (v/v). Control treatment consisted of equivalent amounts of ethanol and peanut oil as contained in the α-TEA treatment. The mixtures were vortexed vigorously then stored at 4° C until used. α-TEA /peanut oil mixture was brought to room temperature and administered by gavage 100 µl/mouse per day. This corresponds to a final concentration of 5 mg α-TEA/mouse/day.

### EXAMPLE 9

### Preparation of liposomal composition containing 2,5,7,8-tetramethyl-(2R-(4R,8R,12-trimethyltridecyl) chroman-6-yloxy) acetic acid (α-TEA)

An α-TEA/liposome ratio of 1:3 (w/w) was determined empirically to be optimal by methods previously described (17). To prepare the α-TEA/lipid combination, the components were first brought to room temperature. The lipid [1,2-dilauroyl-sn-glycero-3-phosphocholine (DLPC); Avanti Polar-Lipids, Inc., Alabaster, AL] at a concentration of 120 mg/ml was dissolved in tertiary-butanol (Fisher Scientific, Houston, TX) then sonicated to obtain a clear solution. α-TEA at 40 mg/ml was also dissolved in tertiary-butanol and vortexed until all solids were dissolved. The two solutions were then combined in equal amounts (v:v) to achieve the desired ratio of 1:3 α-TEA/liposome, mixed by vortexing, frozen at -80° C for 1-2 h, and lyophilized overnight to a dry powder prior to storing at -20°C until needed. Each treatment vial contained 75 mg α-TEA.

### EXAMPLE 10

### Aerosolization and administration of liposome containing 2.5.7.8-tetramethyl-(2R-(4R,8R,12-trimethyltridecyl) chroman-6-yloxy) acetic acid (α-TEA)

Aerosol was administered to mice as previously described (17). Briefly, an air compressor (Easy Air 15 Air Compressor; (Precision Medical, Northampton, PA) producing a 10L/min airflow was used with an AeroTech II nebulizer (CIS-US, Inc. Bedford, MA) to generate aerosol. The particle size of α-TEA liposome aerosol discharged from the AeroTech II nebulizer was determined by the Anderson Cascade Impactor to be 2.01 µm mass median aerodynamic diameter (NMAD), with a geometric standard deviation of 2.04. About 30% of such particles when inhaled will deposit in the respiratory tract of the mouse and the remaining 70% will be exhaled (17).

Prior to nebulization, the α-TEA/lipid powder was brought to room temperature then reconstituted by adding 3.75 ml distilled water to achieve the final desired concentration of 2 0 mg/ml α-TEA. The mixture was allowed to swell at room temperature for 30 min with periodic inversion and vortexing, and then added to the nebulizer. This α-TEA formulation can be administered orally by gavage at levels of 4 mg α-TEA/0.1 ml. Mice were placed in plastic cages (7 x 11 x 5 in.) with a sealed top in a safety hood. Aerosol entered the cage via a 1 cm accordion tube at one end and discharged at the opposite end, using a one-way pressure release valve. Animals were exposed to aerosol until all α-TEA/liposome was aerosolized, approximately 15 min.

### EXAMPLE 11

### Aerosol Characteristics of α-TEA Incorporated into Liposomes

HPLC analyses were conducted on α-TEA liposomes recovered from aerosol in the All Glass Impinger (Ace Glass Co., Vineland, NJ). The delivered dosage = concentration (µg/L) x mouse minute volume (1-min/kg) x duration of delivery (min) x estimated deposited fraction (30%; 17). Based on this formula, we estimate that approximately 36 µg g of α-TEA was deposited in the respiratory tract of each mouse each day (316.2 µg g/L x 1 min/kg x 15 min x 0.30 = 1,422.9 µg g/kg/day.

### EXAMPLE 12

### HPLC Analyses of α-TEA in Tumors

Tumors were removed during necropsy and half of each tumor was quick frozen in liquid nitrogen then stored at -70°C until HPLC analyses were performed. Tumors were processed for HPLC analyses by homogenization and hexane extraction of lipids. Briefly, weighed tumors were placed in 5 ml disposable conical-bottom tubes (Sarstedt, Newton, NC) along with 5-7 Kimble solid glass beads (4mm), 1 ml 1% SDS in water, 1-2 ml 100% ethanol and 1 ml hexane. Samples were then mixed using a Crescent Wig-L-Bug (model 3110B Densply International, Elgin, IL) twice for 1 min each. Samples were then centrifuged for 5 min at 1,000 rpm, and the organic layer collected, the aqueous layer was mixed with hexane again, vortexed, centrifuged, and processed twice more before the organic layer was dried down under nitrogen. Samples were run immediately. Reverse phase HPLC analyses with fluorometric detection of the tocopherol ether analog were conducted as described by Tirmenstein, M.A., et al. (23).

### EXAMPLE 13

### Lung and lymph node Metastastes

Metastastic lesions in the five lung lobes were counted visually at time of sacrifice. Fluorescent green micro-metastatic cancer cell colonies in the left lung lobe and lymph nodes were counted using a Nikon fluorescence microscope (TE-200) with a 20 x objective (200 x magnification) and Image-Pro Plus (version 4.1; Media Cybernetics, Silver Spring, MD) software associated with the microscope. Fluorescent lesions were separated into three size grouping: <5 µm , 5-10 µm and >10 µm.

### EXAMPLE 14

### TUNEL Assay for Detection of Apoptosis in Vivo

Deparaffinized sections (5 µm) of tumor tissue were used to assess apoptosis using reagents supplied in the ApopTag *In Situ* Apoptosis Detection kit (Intergen, Purchase, NY) according to the manufacturer's instructions. Nuclei that stained brown were scored as positive for apoptosis and those that stained blue were scored as negative. At least sixteen 400X microscopic fields were scored per tumor. Data are presented as the mean ± S. E number of apoptotic cells counted in three separate tumors from each group. Pictures of tumor tissue were taken at 1000X magnification.

### EXAMPLE 15

### H&E Staining of Tumor Tissue

Tumors were fixed with 10% neutral buffered formalin, and embedded in paraffin according to standard histological procedures. H&E stained 5 µm thick sections were used to examine tumor morphology.

### EXAMPLE 16

### Histological Evaluation of 66 cl.4-GFP Cells

The murine mammary tumor cells were characterized as spindle cell carcinomas, poorly differentiated, with high mitototic index.

### EXAMPLE 17

### Statistical Analyses

Statistical analyses were conducted using Prism software version 3.0 (Graphpad, San Diego, CA). Animal numbers for experiments were determined by power calculation. Animal weights and tumor volumes were analyzed by student t-test.

### EXAMPLE 18

### Evaluation of anticancer properties of natural and synthetic tocopherols, tocotrienols and derivatives

Balb/c mice were injected with 200,000 66 cl.4 GFP cells subcutaneous in the mammary fat pad area between the 4^{th} and 5^{th} nipple on the right side of the body. 9 days after injection, animals were split into groups (10 animals per group) with comparable size tumors ranging from 0.5 X 0.5 mm -1X2 mm. Animals were treated with liposomal formulations of natural alpha and gamma tocopherols, natural alpha-tocotrienol, tocotrienol enriched fraction (TRF; this fraction contains approximately 32% alpha-tocopherol, 20% alpha tocotrienol, 31% gamma tocotrienol and 12% delta-tocotrienol; Gould, M. N. (30), synthetic dl-alpha-tocopherol, and synthetic derivative dl-alpha-tocopherol acetate by aerosol delivery for 19 or 21 days. Animals were given 75 mg of each compound via nebulization per day which delivered 36 µg of each compound to each animal per day. Animals were palpated every other day and volumes were calculated as (w² x 1)/2.

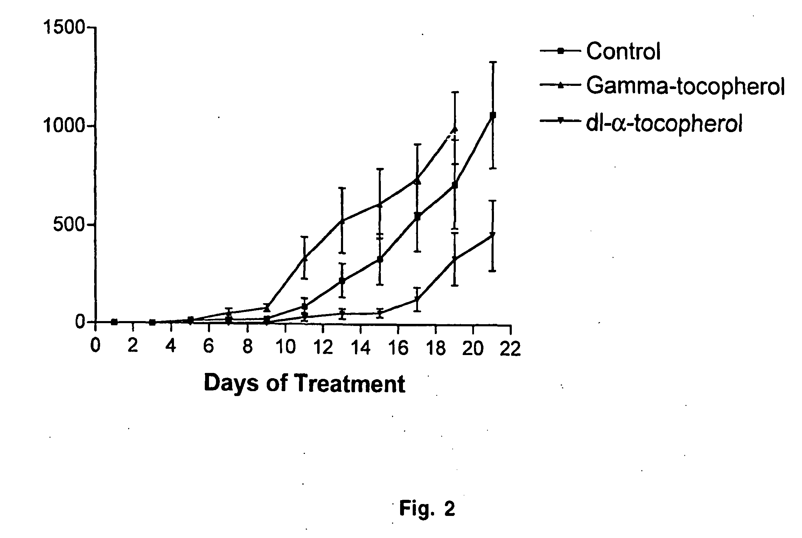

Data is shown only for RRR-gamma-tocopherol and dl-alpha-tocopherol (Figure 2). dl-alpha tocopherol liposomal formulation delivered by aerosol significantly inhibited the growth of 66 cl.4GFP cells by 81 and 73% on days 15 and 17 of treatment, respectively. Although not as effective as dl-alpha-tocopherol, dl-alpha-tocopherol acetate, RRR-delta-tocopherol, RRR-alpha-tocotrienol, tocotrienol rich fraction and (TRF), inhibited tumor growth by 29, 25, 34, 25, and at days 15 and by 57, 40, 27, and 40% at day 17, respectively.

RRR-α-tocopherol and RRR-γ-tocopherol liposomal preparations delivered by aerosol enhanced tumor growth. The enhancement of tumor growth, determined by tumor volume, by RRR-α-tocopherol was not significantly greater than tumor growth in controls; however, tumor volume of mice receiving RRR-γ-tocopherol was significantly higher than the tumor volume of control mice (Figure 2).

Table 2 depicts the percent inhibition of visible lung metastases with these liposomal formulations of vitamin E or its derivatives. These data show that dl-a-tocopherol liposomal formulation delivered by aerosol significantly reduced the number of visible lung metastatic lesions in comparison to controls.

**TABLE 2**

| Treatment | Visible lung metastases/animal | Inhibition (%) |
|---|---|---|
| Control | 1.7 | 0 |
| dl-α-tocopherol | 0.1 | 94** |
| dl-α tocopherol acetate | 0.3 | 82 |
| RRR-delta tocopherol | 0.3 | 82 |
| RRR-α tocopherol | 1.7 | 0 |
| RRR-γ tocopherol | 1.15 | 32 |
| RRR-α-tocotrienol | 0.4 | 77 |
| TRF | 1.0 | 41 |

| | | |
|---|---|---|
| ** significantly enhanced above controls | | |

Immunohistochemical analyses of tumor sections from mice treated with dl-alpha-tocopherol liposomal formulation delivered by aerosol showed that this form of vitamin E is inhibiting tumor growth by reducing tumor blood vessel numbers by 51%, inducing apoptosis by 44%, and reducing cell proliferation by 33%. Table 3 lists mechanisms whereby dl-alpha-tocopherol liposomal formulation delivered by aerosol inhibited growth of 66 cl.4-GFP tumors.

**TABLE 3**

| Mechanisms of growth inhibition by dl-alpha-tocopherol liposomes | | | | |
|---|---|---|---|---|
| Treatment | | CD31 Blood Vessel Nos/Area | TUNEL Positive Apopotic Cells/Area | KI67 Inhibition of Proliferation (%) |
| Control | | 227 | 1.4 | 60 |
| dl-α-tocopherol | | 112 | 2.5 | 40 |
| | % Reduction | 51% | ---- | 33% |
| | % Enhanement | --- | 44% | --- |

Furthermore, analyses of mechanisms of action of α-TEA in the transplantable, syngeneic mouse mammary cancer model show that α-TEA reduces cell proliferation and induces apoptosis. More specifically, mean values of the proliferation biomarker KI-67, detected by immunochemistry, were significantly reduced by 56% and mean apoptotic values, determined by TUNEL immunohistochemistry, were significantly increased by 30% in comparison to controls (unpublished data). Thus, it is contemplated that these surrogate markers may have relevance as biomarkers for quantitatively and/or qualitatively determining chemoprevention efficacy of α-TEA.

### EXAMPLE 19

### VES and α-TEA Induce Apoptosis in 66 cl.4 GFP cells In Vitro

Previous studies indicate that vitamin E succinate is a potent apoptotic inducer in many human cancer cell lines, including breast cancer. For comparative purposes, we included vitamin E succinate in the *in vitro* analyses of α-TEA induced apoptosis. Balb/c mammary cancer 66 cl.4-GFP cells were treated with vitamin E succinate or α-TEA, and apoptosis was assessed by morphological analyses of DAPI stained cells for condensed nuclei and fragmented DNA.

Nuclei from 66 cl.4-GFP cells treated with 10 µg/ml α-TEA or vitamin E succinate for three days exhibited condensation and fragmented DNA, characteristics of apoptosis; whereas, nuclei from untreated cells did not exhibit these characteristics (Fig. 3A). 66 cl.4 GFP cells treated with 2.5, 5, 10, and 20 µg/ml α-TEA or vitamin E succinate for three days exhibited dose dependent apoptosis of 5, 6, 34, and 50 % apoptosis for α-TEA, and 3, 5, 16, and 34% apoptosis for vitamin E succinate (Fig. 2B). Untreated, VEH, and EtOH controls exhibited background levels of apoptosis of 2, 2, and 3%, respectively (Fig. 3B).

α-TEA was shown to induce apoptosis in a time-dependent manner. 66 cl.4 GFP cells treated with 10 µg/ml α-TEA for 2-5 days exhibited 20, 35, 47, and 58% apoptosis, respectively (Fig. 3C). Induction of apoptosis was confirmed by the presence of PARP cleavage following treatment of 66 c1.4-GFP cells with 5, 10, and 20 µg/ml α-TEA for 48 hours (Fig. 3D). The 84 kDa cleavage fragment of PARP was evident at both 10 and 20 µg/ml α-TEA treatment; whereas, only intact PARP protein was detected in cells treated with 5 µg/ml α-TEA or in the untreated control cells (Fig. 3D).

### EXAMPLE 20

### Induction of Apoptosis by α-TEA in Vivo

In view of the *in vitro* data showing that α-TEA inhibits 66 cl.4-GFP tumor cell growth via induction of apoptosis, three tumors from each of the liposomal α-TEA/aerosol treatment and aerosol control groups were examined for apoptosis using TUNEL staining of 5 micron tumor sections. Tumors from mice treated with α-TEA had a mean ± S. E. of 2.04±0.23 apoptotic cells/field; whereas, tumors from aerosol control mice had a mean ± S.E. of 0.67±0.15 apoptotic cells/field (p < 0.03; Figure 4A). Positive stained apoptotic cells in tumor sections from liposomal α-TEA aerosol and control treated mice can be seen in Figure 4B.

### EXAMPLE 21

### α-TEA Inhibits 66 cl.4-GFP Clonal Growth

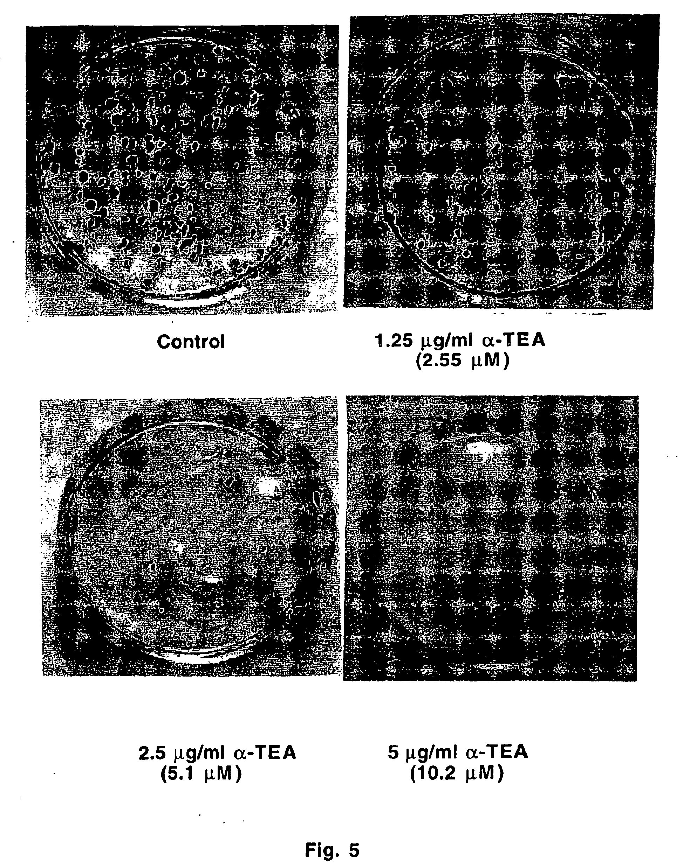

α-TEA at 1.25, 2.5, and 5 µg/ml decreased colony formation by 30, 85, and 100 % when compared to EtOH control (Fig. 5). Untreated (data not shown) and EtOH controls averaged 146 ± 11 S. D. and 140 ± 22 S. D. colonies, respectively. Cells treated with α-TEA at 1.25 and 2.5 µg/ml averaged 98 ± 20 S. D. and 21 ± 6 S. D. colonies, respectively. No colonies formed when cells were treated with α-TEA at 5 (Fig. 5) or 10 µg/ml.

### EXAMPLE 22

### Effect of α-TEA liposomal aerosol treatment on body weight

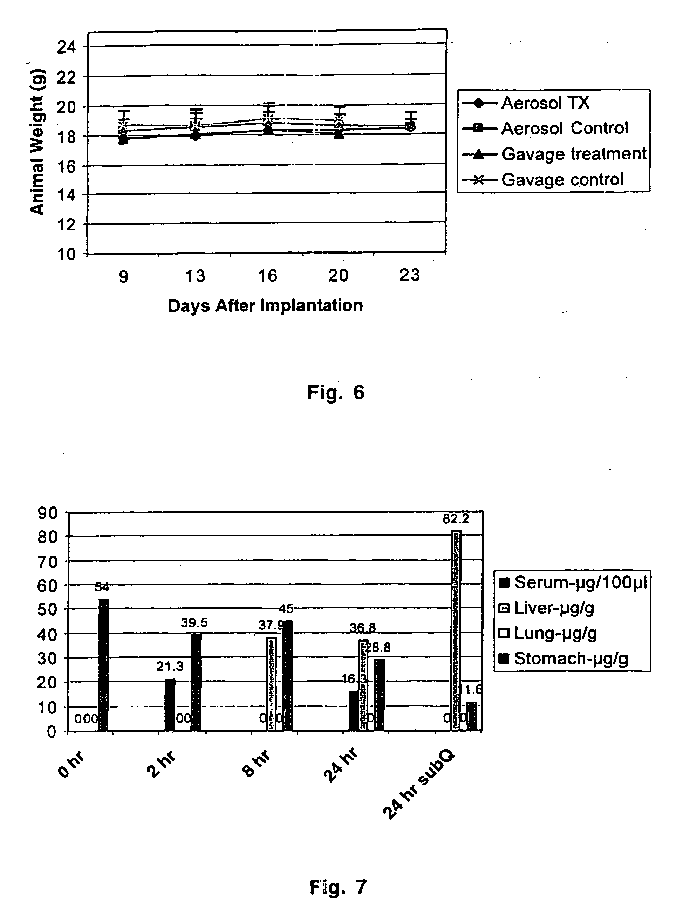

Balb/c mice, 10/group (4 groups, 40 mice total) are inoculated with 200,000 66 cl.4 GFP cells as described on day 0. Nine days after tumor inoculation, treatments groups are initiated: Aerosol Treatment (TX) = aerosol delivery of α-TEA liposome (5 mg/mouse)/daily through day 23. Aerosol Control = Aerosol delivery of liposome composition only daily through day 23. Gavage treatment = gavage administration of α-TEA at 5 mg/mouse in ethanol/peanut oil daily through day 23. Gavage control = gavage administration of ethanol/peanut oil only through day 23. Mice were weighed at the initiation of treatment (day 9) and thereafter on days 13, 16, 20, and 23 (Fig. 6). Data are presented as the mean +/- standard deviation. There are no significant weight differences in the four groups.

### EXAMPLE 23

### Effect of α-TEA liposomal aerosol treatment on serum and tissue levels

Eight mice are treated via pulmonary aerosol delivery with 40mg of α-TEA (5 mg/mouse) in 6 ml liposomes. The mice inhaled the aerosol over a period of 30 minutes until delivery was complete. Mice are sacrificed 0, 2, 6, or 24 hours after the treatment ended. Serum and tissue levels of α-TEA are determined by HPLC analyses (Fig. 7). At the first sacrifice (time 0), α-TEA is found only in the stomach tissue. α-TEA is present in the serum and stomach of mice sacrificed at 2 hours after completion of treatment. α-TEA is present in the liver and stomach at 6 hours after completion of treatment. α-TEA is present in the liver and stomach at 24 hours after completion of treatment.

### EXAMPLE 24

### Effect of α-TEA liposomal aerosol treatment on tumor weight

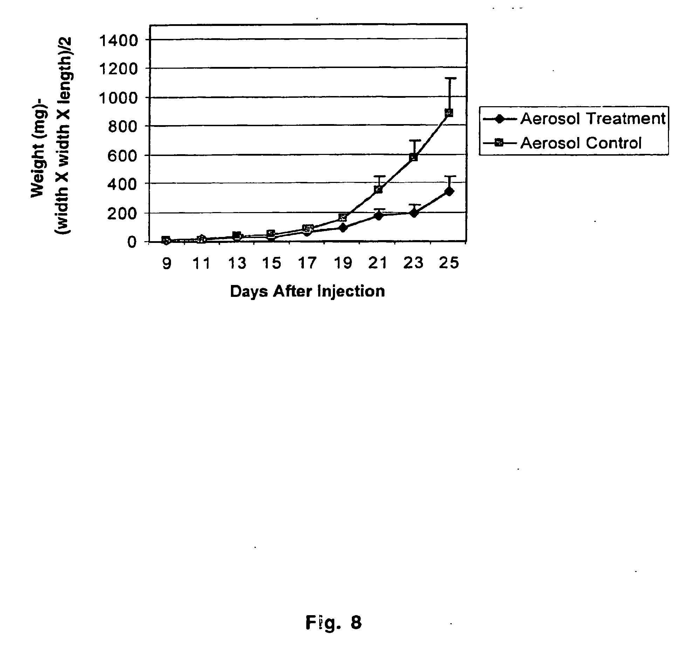

Mice are injected with 200,000 66cl.4 GFP murine mammary tumor cells as described on day 0. Treatments are initiated on day 9 when tumors reached 1-3 mm in size. Aerosol treatments of α-TEA liposomes (5 mg α-TEA /mouse) are administered daily for 16 days (Fig. 8). Data represent the mean +/- Standard Error (SE), N = 10 mice for control and treatment groups. At the time of sacrifice on days 25, 16 after treatment initiation, the size of tumors in the aerosol α-TEA liposomes treatment group are 61% less than the tumor size of the aerosol only control group.

### EXAMPLE 25

### Liposomal α-TEA/Aerosol Treatment Suppressed 66 cl.4-GFP Tumor Growth in Balb/c Mice and Reduced Lung Micrometastases

Balb/c mice were injected s. c. with 2 X 10⁵ 66 cl.4-GFP cells in the inguinal area between the 4^{th} and 5^{th} nipple on the right side of the body. When tumors reached 2 x 2-4 x 4 mm (9 days after tumor injection), mice were placed into 5 groups ((Group 1: untreated control, Group 2: liposome/aerosol control, Group 3: liposomal α-TEA aerosol treatment, Group 4: peanut oil/gavage control, Group 5: α-TEA in peanut oil/gavage treatment) of 10 mice/group) such that the mean tumor volume of each group was closely matched. Daily treatments were initiated on day 9 after tumor injection.

The mean tumor volume of the liposomal α-TEA/aerosol treatment group, in comparison to aerosol control, was reduced by 23, 41, 50, 67 and 61 % for days 9, 11, 13, 15, and 17 of treatment, respectively (Fig. 9A). At sacrifice, lungs were taken, examined visually for metastatic lesions and frozen for analyses of micrometastases by fluorescence. No visible tumors were seen in the α-TEA treatment group; whereas, the untreated and aerosol control groups exhibited 3.25±1.7 and 4.25±0.5 visual tumors/animals exhibiting lung metastases, respectively, as shown in Table 4.

Use of a Nikon fluorescence microscope and Image-Pro Plus software permitted measurement of green fluorescing micrometastases into three size groupings of <5 µm, 5-10 µm and >10 µm. This analysis showed a highly significant decrease in tumor metastases of all three sizes in the α-TEA treatment group in comparison to the aerosol and untreated controls (Fig. 9B). The mean number of micrometastases in the α-TEA treatment group (11.4 ± 3.5 S. E.; N= 8), in comparison to aerosol control (60.0 ±15 S.E.; N = 10), was reduced by 81 % (p <0.2). Although the mean number of micrometastases in the aerosol control group versus the untreated control (N = 10) was reduced (60±15.2 S. E. versus 101.7± 17.0 S. E.; p <0.9), the differences were not considered to be significant due to the great range in numbers of micrometastases among the mice within these two groups (Fig. 9B).

**TABLE 4**

| 66-cl.4-GFP Mammary Cancer Cell Lung Lung Metastases in Balb/c Mice | | |
|---|---|---|
| Treatments | No. Animals/Group with Visible Lung Metastases^{a} | No. Visible Lung Tumor Foci/Animal^{b} |
| No Treatment | 4 / 10 | 3.25±1.7 |
| Aer./Liposome Control | 4 / 10 | 4.25±0.5 |
| Aer./Liposome/α-TEA | 0 / 10 | 0 |

| | | |
|---|---|---|
| ^{A}Metastatic lesions in all five lung lobes for each animal in all treatment groups were counted visually at the time of sacrifice. ^{b}Data are expressed as the mean ±S.D. of visible lung tumor foci observed in the four lung metastases bearing animals in the two control groups. | | |

### EXAMPLE 26

### Comparison of α-TEA and vitamin E succinate (VES) liposomes delivered via aerosolization on 66 cl.4 GFP tumor growth

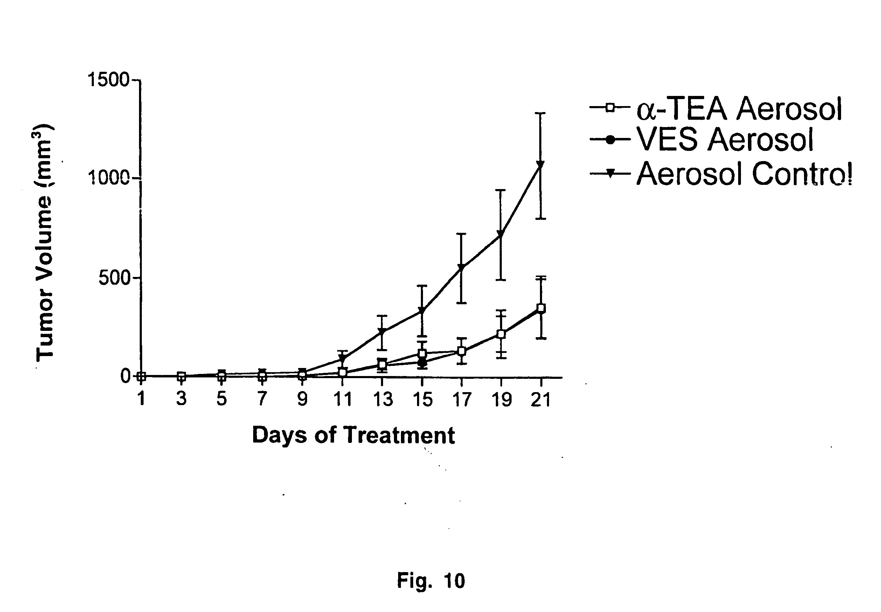

Balb/c mice were injected with 200,000 66 cl.4 GFP cells subcutaneous in the mammary fat pad area between the 4^{th} and 5^{th} nipple on the right side of the body. 9 days after injection, animals were split into groups (10 animals per group) with comparable size tumors ranging from 0.5 X 0.5 mm - 1 X 2 mm. Animals were treated with α-TEA in liposome, vitamin E succinate in liposome, or control liposome alone via aerosol, 7 days per week, for 21 days. Animals were given 75 mg compound via nebulization per day such that each animal received 36 µg compound per day. Animals were palpated every other day and volumes were calculated as (w² x 1)/2. α-TEA liposome aerosol reduced tumor growth by 64, 76, 69, and 67% on days 15,17,19, and 21, respectively (values were statistically significantly different between α-TEA and control on days 17, 19, and 21, p= 0.03, 0.048, and 0.03, respectively). VES liposome aerosol reduced tumor growth by 76, 76, 69, and 68% on days 15,17,19, and 21, respectively (Fig. 10). Values were statistically significantly different between vitamin E succinate and control on days 17, and 21, p= 0.029, and 0.029 respectively.

### EXAMPLE 27

### Effect of gavage delivery of α-TEA on tumor weight

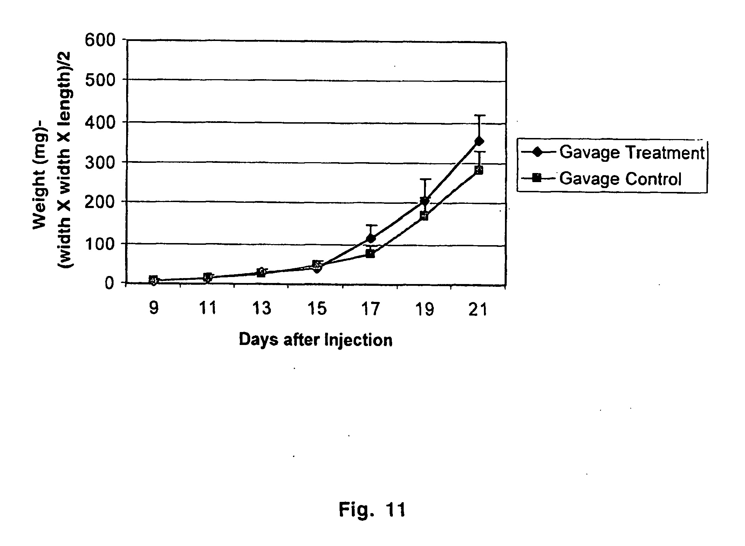

Delivery of α-TEA by gavage is ineffective in preventing the growth of 66 cl.4 GFP murine mammary tumor cells at the inoculation site (Fig. 11). The size of the tumors, 9 - 21 days after injection of 200,000 66 cl.4 GFP cells between the 4^{th} and 5^{th} nipple on the right side of each mouse, is determined. Treatments are initiated on day 9 after tumor inoculation. Each group consists of n=10 +/- SE. Gavage Treatment = Treatments of 5 mg of α-TEA in ethanol and peanut oil (0.1 ml volume) are administered daily starting on day 9 after tumor inoculation and continuing through day 21. Gavage Control = Mice received peanut oil and ethanol alone (0.ml volume)/daily, starting on day 9 after tumor inoculation and continuing through day 21. Data (tumor size) are depicted as the mean +/- standard error for days 9, 11, 13, 15, 17, 19, and 21. There are no significant differences in tumor weights between the control and gavage/α-TEA treatment groups at all time points.

### EXAMPLE 28

### Delivery of α-TEA by Gavage Did Not Reduce Tumor Growth at Inoculation Site But Reduced Lung Micrometastases

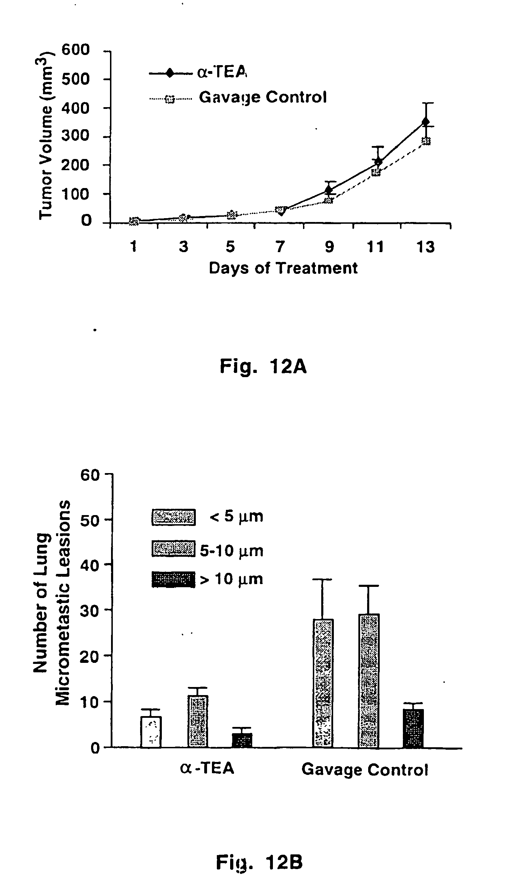

In contrast to liposomal α-TEA/aerosol treatment, mean tumor volumes from mice receiving 5 mg/day/mouse α-TEA EtoH/peanut oil formulation administered by gavage did not differ from the mean tumor volume of the gavage control (Fig. 12A). However, administration of α-TEA by gavage reduced the number of lung tumor micrometastases by 68%. The numbers of micrometastases, based on three size groupings (< 5 µm, 5-10 µm, >10 µm), were 6.8±1.5, 11.3± 1.8 and 3.1±1.2 S. E. for mice administered α-TEA by gavage; whereas, micrometastases in control mice were 27.9±9.0. 29.2± 6.3, and 8.4±1.5 S. E., respectively (Fig. 12B).

No differences in mean body weights among any of the treatment or control groups were observed (data not shown). Non-tumor bearing mice that were treated with either aerosol/α-TEA or gavage for 17 days and then kept for eleven months to assess long term effects did not show any adverse effects of the α-TEA treatments.

Although administration of α-TEA by aerosol was superior to administration by gavage in that α-TEA administered by gavage did not reduce tumor size at the site of inoculation in comparison to tumor size of control mice, it is of interest that the number of lung micrometastases were reduced in comparison to control when α-TEA was administered by gavage. This suggests that α-TEA was bioavailable. Since α-TEA is non-hydrolyzable, and expectations are that when delivered by gavage, it should be an effective anti-tumor agent, it is contemplated that α-TEA administered at 5 mg/ml/day/mouse was not effective in reducing tumor growth at the site of tumor inoculation due to low uptake via the digestive tract. Thus, it is further contemplated that low levels of α-TEA may be effective in preventing lung tumor foci from being established.

### EXAMPLE 29

### Comparison of α-TEA/gavage, liposomal α-TEA/gavage and liposomal α-TEA/aerosol inhibition of tumor growth and lung and lymphnode metastases of 66 cl.4 mammary tumors

Although effective in inhibiting lung micrometastases, an α-TEA/EtOH/peanut oil formulation administered by gavage does not inhibit tumor growth in the transplantable syngeneic mammary cancer animal model. An α-TEA/liposomal formulation administered orally by gavage twice a day at 6 mg α-TEA/day, however, is an effective method of delivery for prevention of cancer growth as well as metastases of 66 cl.4 Balb/c mammary tumor cells in female Balb/c mice. The liposomal formulation of α-TEA delivered by gavage inhibits tumor growth by 70% and inhibits lung and lymphnode metastases by 59% and 56%. It is estimated that 36 µg of α-TEA is deposited in lungs of mice/day when α-TEA/liposomal preparations are administered by aerosol. The amount of α-TEA deposited in tissues when α-TEA/liposomal or α-TEA/EtOH/peanut oil formulations are given by gavage is not known. A comparison of anti-tumor efficacy of treatment regimes is shown in Table 5.

**TABLE 5**

| Comparison of α-TEA Formulations and Methods of Administration In Prevention of Tumor Growth and Metastases of 66 cl.4 Balb/c mammary tumor cells | | | | | |
|---|---|---|---|---|---|
| | | | Inhibition (%) | | |
| Formulation | Delivery Method | α-TEA Conc (per mouse) | Tumor Growth | Lung | Metastases Lymphnode |
| Liposomal | Gavage | 3 mg/2Xd | 70% | 59% | 56% |
| Liposomal | Aerosol | 5 mg/d | 70% | 81% | 94% |
| EtOH/P Oil | Gavage | 5 mg/d | 0% | 62% | not tested |

### EXAMPLE 30

### Comparison of α-TEA and vitamin E succinate liposomes delivered via gavage on 66 cl.4 GFP tumor growth

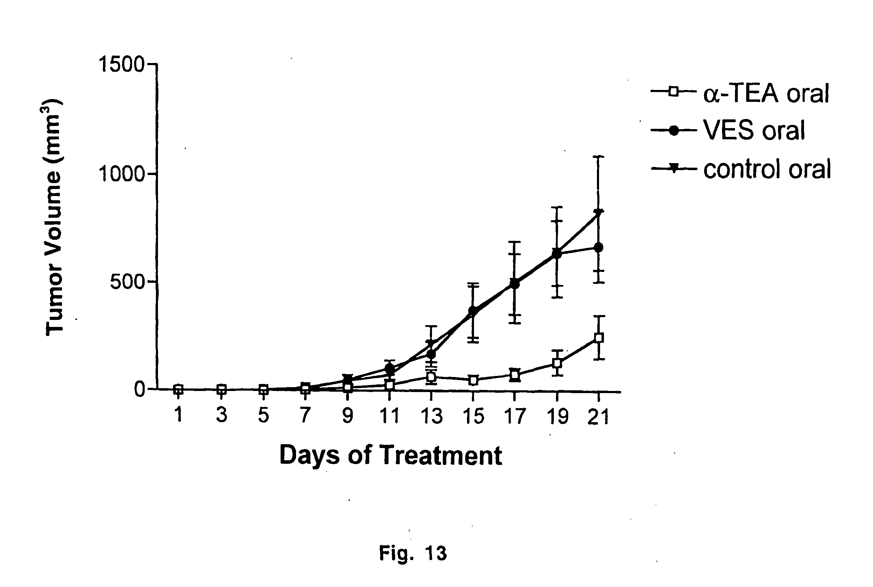

α-TEA liposomal formulation delivered orally b y gavage inhibited growth of murine mammary cancer cells transplanted into Balb/c mice. Vitamin E succinate liposomal formulation delivered orally by gavage did not inhibit growth of murine mammary cancer cells transplanted into Balb/c mice. Balb/c mice were injected with 200,000 66 cl.4 GFP cells subcutaneous in the mammary fat pad area between the 4^{th} and 5^{th} nipple on the right side of the body. 9 days after injection, animals were split into groups (10 animals per group) with comparable size tumors ranging from 0.5 X 0.5 mm - 1 X 2 mm. Animals were treated with α-TEA in liposome, vitamin E succinate in liposome, or control liposome alone via oral gavage, 2 times per day, 7 days per week, for 21 days. Animals were given a total of 6 mg compound per day. Animals were palpated every other day and volumes were calculated as (w² x 1)/2. α-TEA liposome gavage reduced tumor growth by 85, 85, 80, and 70% on days 15,17,19, and 21, respectively (values were statistically significantly different between α-TEA and control on days 15, 17, and 19 (p= 0.03, 0.039, and 0.029, respectively). VES liposome gavage did not reduce tumor growth (Fig. 13).

### EXAMPLE 31

### Liposomal α-TEA gavage treatment reduced 66 c1.4-GFP lymphnode and lung micrometastases

Balb/c mice were injected with 200,000 66 cl.4 GFP cells subcutaneous in the mammary fat pad area between the 4^{th} and 5^{th} nipple on the right side of the body. 9 days after injection, animals were split into groups (10 animals per group) with comparable size tumors ranging from 0.5 X 0.5 mm - 1 X 2 mm. Animals were treated with α-TEA in liposome, vitamin E succinate in liposome, or control liposome alone via oral gavage, 2 times per day, 7 days per week, for 21 days. Animals were given a total of 6 mg compound per day. At sacrifice, the left lung lobe of each mouse of flash frozen and saves for detection of GFP expressing micrometatstic lesions via fluorescent microscopy.

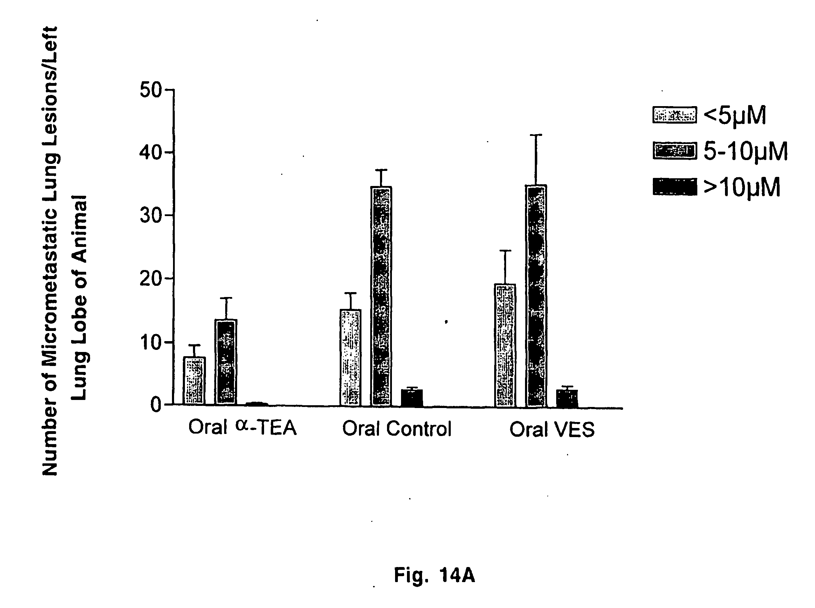

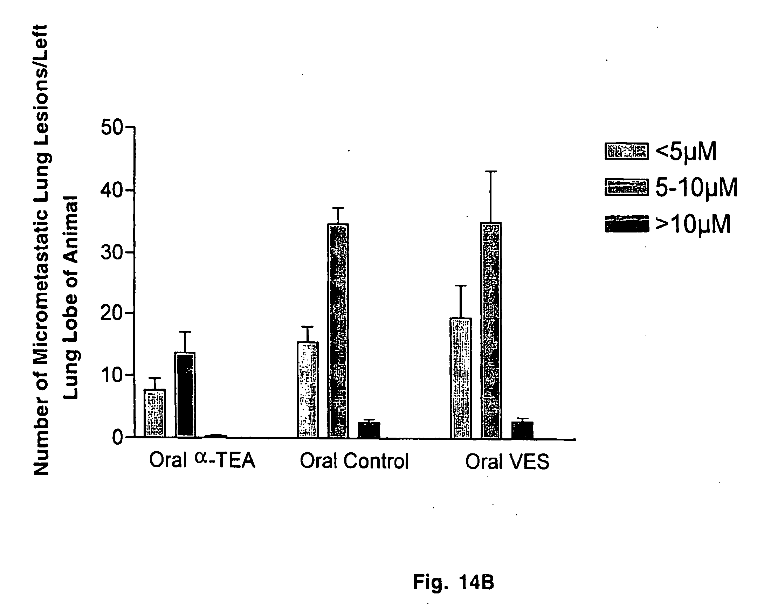

Animals given α-TEA liposomal formulation via gavage, showed a marked decrease in micrometatstatic lung lesions compared to control (21.5/lung vs. 52.7/ lung, respectively). Vvitamin E succinate liposomal formulation **via gavage** had no effect on lung metastasis (Fig. 14A). At sacrifice, lymph nodes were collected and viewed for micrometastatic lung lesions. Animals given α-TEA via gavage showed lymph nodes with an average of 3.1 ±0.8 miceo metastatic lesions vs 7.1 ±1.7 micrometastatic lesions in the control group (p=0.036). Vitamin E succinate via gavage was not effective in decreasing micrometastatic lung lesions (Fig. 14B).

### EXAMPLE 32

### Liposomal α-TEA gavage treatment reduced tumor growth of MDA-MB-435 breast cancer cells in vivo

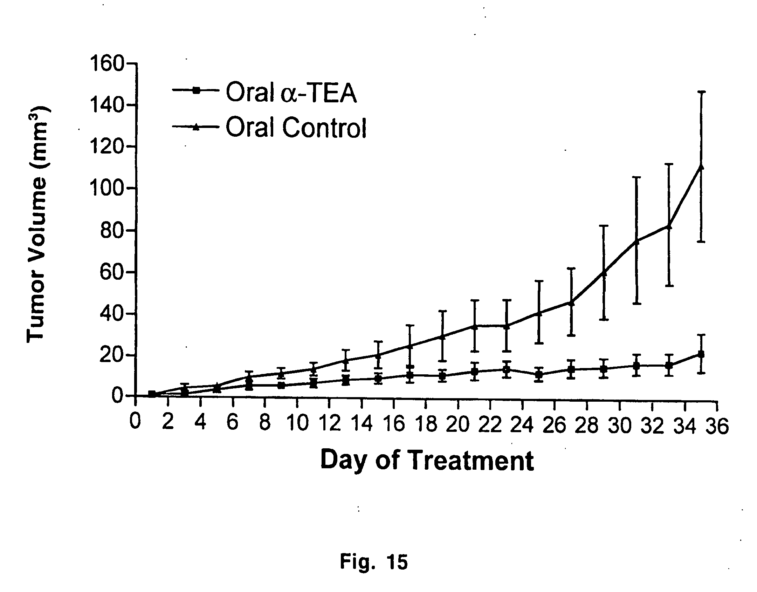

Nu/Nu athymic nude mice were injected with 1.0 X 106 MDA-MB-435 GFP FL cells subcutaneous in the mammary fat pad area between the 4^{th} and 5 th nipple on the right side of the body. 8 days after injection, animals were split into groups (10 animals per group) with comparable size tumors ranging from 0.5 X 0.5 mm - 2 X 2 mm. Animals were treated with alpha-TEA in liposome, or control liposome alone via oral gavage, 2 times per day, 7 days per week, for 21 days. Animals were given a total of 8 mg compound per day. Animals were palpated every other day and volumes were calculated as (w² x 1)/2. Alpha-TEA liposome gavage reduced tumor growth by 68, 75, 78, 79 and 79% on days 27, 29, 31, 33, and 35, respectively (Fig. 15). Values were statistically significantly different between α-TEA and control on days 33 and 35 (p= 0.045 and 0.033, respectively).

### EXAMPLE 33

### Inhibition of tumor growth and lung metastases of 66 cl.4-GFP tumors in Balb/c Mice by aerosol liposomal/α-TEA+9NC combination

Female Balb/c mice of 6 weeks of age were subcutaneously injected with 200,000 66 clone 4-GFP mammary tumor cells between the 4th and 5^{th} nipples on the right side. When tumors reached 1 x 1 millimeters in size, aerosol treatments were initiated. α-TEA administered separately and in combination with low levels, 0.403 µg/day of 9-nitrocamptothecin is a potent inhibitor of tumor growth. Aerosol delivery of α-TEA + 9-nitrocamptothecin inhibited murine mammary tumor growth by 90%; whereas, α-TEA and 9-nitrocamptothecin alone inhibited tumor growth by 65% and 58%, respectively. Aerosol delivery of α-TEA + 9-nitrocamptothecin inhibited axillary lymph node and lung micrometastatic lesions by 87% and 71%; whereas, α-TEA and 9-nitrocamptothecin administered alone by aerosol inhibited lymph node micrometastatic lesions by 94% and 60%, and inhibited lung micrometastatic lesions by 71% and 55%, respectively. Thus, α-TEA + 9-nitrocamptothecin administered sequentially inhibits tumor growth better than when the two drugs are given separately. α-TEA separately is as effective in inhibition of lung and lymphnode metastases as when administered in combination with 9-nitrocamptothecin. A comparison of data when α-TEA is administered separately and in combination with 9-nitrocamptothecin in the prevention of tumor growth and metastases is provided below in Table 6.

**TABLE 6**

| Comparison of α-TEA Separately and In Combination with 9-Nitrocamptothecin (9NC) in Prevention of Tumor Growth and Metastases | | | | | |
|---|---|---|---|---|---|
| | | | Inhibition (%) | | |
| | Delivery | Drug Conc. | Tumor | Metastases | |
| Formulation | Method | In Nebulizer (per mouse) | Growth | Lung | Lymphnode |
| Lip/α-TEA | Aerosol | 5 mg/d | 65% | 71% | 94% |
| Lip/9NC | Aerosol | 2 µg/d | 58% | 55% | 60% |
| Lip/ α-TEA | Aerosol | 5 mg+2µg/d | 90% | 71% | 87% |
| + 9-NC | | | | | |

### EXAMPLE 34

### α-TEA converts cisplatin resistant Cp-70 human ovarian cancer cells to cisplatin sensitive

Combinations of α-TEA + cisplatin enhances tumor cell killing *in vitro* and *in vivo.* The cp-70 cell line, a human ovarian cancer, shows resistance to cisplatin, a platinum drug that is commonly used as a first-line treatment for ovarian tumors. The cp-70 sub-clone was developed *in vitro* from the A2780 cisplatin-sensitive cell line through intermittent exposure to increasing levels of cisplatin, up to 70 mM. When treated in culture with α-TEA, both cell lines A2780 and cp-70 undergo apoptosis (Fig. 16A). Treatment of cp-70 human ovarian cancer cells with sub-optimal levels of cisplatin (0.625 and 1.25 µg/ml) and α-TEA (10 µg/ml) restores cisplatin sensitivity to cp-70 cells, increases the levels of apoptosis in the A2780's and restores cisplatin-sensitivity in the cp-70 cell line (Fig. 16B). α-TEA in combination with cisplatin inhibits growth of cp-70 cells *in vivo.* The gene for green-fluorescent protein (GFP) was expressed in cp-70 cells via viral infection so that the cells would glow and could be used to study co-treatment effects in a xenograft mouse model. The combination treatment of cisplatin and α-TEA is tested in an in *vivo* xenograft nude mice model. In this model, female nude mice were inoculated with 1 x 10⁶ cells subcutaneously between the 4^{th} and 5^{th} nipples. When the average tumor size reached 1.0 mm³, the mice were selected for treatment with either α-TEA alone, delivered via aerosol in liposomes, cisplatin alone (5mg/kg) injected I.P. once per week for 3 weeks, cisplatin + α-TEA, or aerosol control (liposome only). Data show α-TEA + cisplatin to be an effective method for inhibiting the growth of cisplatin resistant cp-70 human ovarian cancer cells (Fig. 16C).

### EXAMPLE 35

### Effect of alpha-TEA, methylselenocysteine and trans-resveratrol on MDA-MB-435-GFP-FL breast cancer cells

Female NU/NU homozygous mice were injected with one million MDA-MB-435 GFP FL cells subcutaneously in the mammary fat pad area between the 4^{th} and 5^{th} nipples on the right side of the body. Nine days after injection, animals were split into five groups of ten animals with comparable sized tumors ranging from 0.5mm x 0.5 mm to 2.0mm x 2.0mm. Animals were gavaged 3ppm methylselenocysteine (MSC) in water, α-TEA in liposome via aerosol, or gavaged 10 mg/kg b.w. *trans*-resveratrol (*t*-RES) in first 1:1, EtOH: physiological saline, and starting at day 24, 6.5% EtOH and 93.5% Neobee oil. A combination group was treated with each of the above three compounds, and a control group was treated with aerosolized liposome, 100 µl water, and 50 µl of the *t-*Res solvent. Animals were treated seven days a week, for 35+ days. Tumors were palpated every other day and volumes were calculated using the formula (w² x 1)/2.

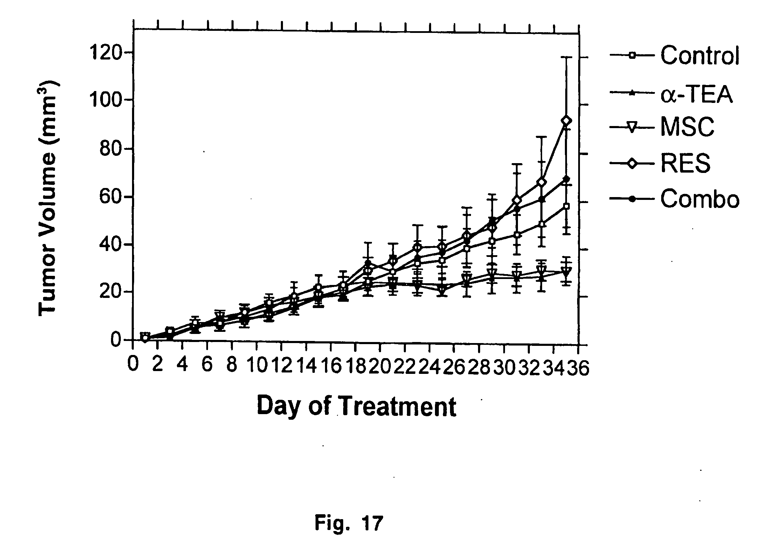

At day 35, the α-TEA group showed a 47.8% decrease, the MSC group a 47.2% decrease, the *t*-RES group a 61.2% increase, and the combination group a 19.8% increase from the control (Fig. 17). The groups were statistically analyzed to the control with a t-test and the p values were p=0.0487, 0.0347, 0.2221, and 0.6255 for the α-TEA, MSC, *t*-RES, and combination group respectively. Thus, only the alpha-TEA and MSC groups tested separately gave reduced tumor growth significantly different from the controls. Trans-resveratrol only treated and the combination of the three compounds enhanced tumor growth.

The following references are cited herein:
1. Prasad, et al., Effects of tocopherol (vitamin E) acid succinate on morphological alterations and growth inhibition in melanoma cells in culture. Cancer Res., 42: 550-554, 1982.
2. Prasad, et al., Vitamin E and cancer prevention: Recent advances and future potentials. J. Am. Coll. Nutr., 11: 487-500, 1992.
3. Schwartz, J., and Shklar, G. The selective cytotoxic effect of carotenoids and α-tocopherol on human cancer cell lines in vitro. J. Oral Maxillofac. Surg., 50: 367-373, 1992.conserved homolog, Bax, that accelerates programmed cell death. Cell, 74: 609-619, 1993.
4. Fariss, et al., The selective antiproliferative effects of α-tocopheryl hemisuccinate and cholesteryl hemisuccinate on murine leukemia cells result from the action of the intact compounds. Cancer Res., 54: 3346-3351, 1994.
5. Kline, K., Yu, W., and Sanders, B. G. Vitamin E: Mechanisms of action as tumor cell growth inhibitors. In: K. N. Prasad and W. C. Cole (eds.), Proceeding of the International Conference on Nutrition and Cancer, pp 37-53. Amsterdam: IOS Press, 1998.
6. Kline, K., Yu, W , and Sanders, B. G. Vitamin E Mechanisms of action as tumor cell growth inhibitors. J. Nutr., 131: 161S-163S, 2001.
7. Neuzil, J., Weber, T., Gellert, N., and Weber, C. Selective cancer cell killing by α-tocopheryl succinate. Br. J. Cancer, 84: 87-89, 2000.
8. Malafa, M.P., and Neitzel, L. T. Vitamin E succinate promotes breast cancer tumor dormancy. J. Surg. Res., 93: 163-170, 2000.
9. Malafa, et al., Vitamin E inhibits melanoma growth in mice. Surgery, 131: 85-91, 2002.
10. Neuzil, et al., Induction of cancer cell apoptosis by α-tocopheryl succinate: molecular pathways and structural requirements. FASEB J., 15: 403-415, 2001.
11. Weber, et al., Vitamin E succinate is a potent novel antineoplastic agent with high selectivity and cooperativity with tumor necrosis factor-related apoptosis-inducing ligand (Apo2 ligand) in vivo. Clin. Cancer Res., 8: 863-869, 2002.
12. Wu, et al., Inhibitory effects of RRR-alpha-tocopheryl succinate on benzo(a)pyrene (B(a)P)-induced forestomach carcinogenesis in female mice. World J. Gastroenterol., 7: 60-65, 2001.
13. You, H., Yu, W., Sanders, B.G., and Kline, K. RRR-α-tocopheryl succinate induces MDA-MB-435 and MCF-7 human breast cancer cells to undergo differentiation. Cell Growth Differ., 12: 471-480, 2001.
14. You, H., Yu, W., Munoz-Medellin, D., Brown, P. H., Sanders, B. G., and Kline, K. Role of extracellular signal-regulated kinase pathway in RRR-α-tocopheryl succinate-induced differentiation of human MDA-MB-435 breast cancer cells. Mol. Carcinogenesis, 33: 228-236, 2002.
15. Yu, et al., Activation of extracellular signal-regulated kinase and c-Jun-NH2-terminal kinase but not p38 mitogen-activated protein kinases is required for RRR-α-tocopheryl succinate-induced apoptosis of human breast cancer cells. Cancer Res., 61: 6569-6576, 2001.
16. Koshkina, et al., Distribution of camptothecin after delivery as a liposome aerosol or following intramuscular injection in mice. Cancer Chemother. Pharmacol., 44: 187-192, 1999.
17. Knight, et al., Anticancer effect of 9-nitrocamptothecin liposome aerosol on human cancer xenografts in nude mice. Cancer Chemother. Pharmacol., 44: 177-186, 1999.
18. Koshkina, et al., 9-Nitrocamptothecin liposome aerosol treatment of melanoma and osteosarcoma lung metastasis in mice. Clin. Cancer Res,. 6: 2876-2880, 2000.
19. Walrep, et al., Pulmonary delivery of beclomethasone liposome aerosol in volunteers. Chest., 111: 316-23, 1997.
20. Dexter, et al., Heterogeneity of tumor cells from a single mouse mammary tumor. Cancer Res., 38: 3174-3181, 1978.
21. Miller, B. E., Roi, L. D., Howard, L. M., and Miller, F. R Quantitative selectivity of contact-mediated intercellular communication in a metastatic mouse mammary tumor line. Cancer Res., 43: 4102-4107, 1983.
22. Yu, et al., Vitamin E succinate (VES) induces Fas sensitivity in human breast cancer cells: role for Mr 43,000 Fas in VES-triggered apoptosis. Cancer Res., 59: 953-961, 1999.
23. Tirmenstein, et al., Sensitive method for measuring tissue alpha-tocopherol and alpha-tocopheryloxybutyric acid by high-performance liquid chromatography with fluorometric detection. J. Chromatogr. B Biomed. Sci. Appl., 707: 308-311, 1998.
24. Schwenke, D. C. Does lack of tocopherols and tocotrienols put women at increased risk of breast cancer? J. Nutr. Biochem. 13:2-20, 2002.
25. Kamal-Eldin, A. and Appelqvist, L.-A. The chemistry and antioxidant properties of tocopherols and tocotrienols. Lipids 31:671-701, 1996.
26. Waldrep, et al., Operating characteristics of 18 different continuous-flow jet nebulizers with beclomethasone dipropionate liposome aerosol. Chest 105:106-110, 1994.
27. Waldrep, et al., Nebulized glucocorticoids in liposomes: Aerosol characteristics and human dose estimates. J Aerosol Medicine 7:135-145, 1994.
28. Pantazis, P. et al. Regression of human breast carcinoma tumors in immunodeficient mice treated with 9 - nitrocamptothecin: differential response of nontumorigenic and tumorigenic human breast cells in vitro. Cancer Research 53:1577-82, 1993.
29. Chatterjee, D. et al. Induction of apoptosis in 9-nitrocamptothecin-treated DU145 human prostate carcinoma cells correlates with de novo synthesis of CD95 and CD95 ligand and down-regulation of cFLIP(short). Cancer Research 61:7148-7154, 2001.
30. Yu, W. et al. RRR-_-tocopheryl succinate-induction of DNA synthesis arrest of human MDA-MB-435 cells involves TGF-α_ independent activation of p21 (Waf1/Cip1). Nutrition and Cancer In Press.
31. Gould, N. et al. A comparison of tocopherol and tocotrienol for the chemoprevention of chemically induced rat mammary tumors. Am. J. Clin. Nutr. 53: 1068S-1070S, 1991

Any patents or publications mentioned in this specification are indicative of the levels of those skilled in the art to which the invention pertains.

One skilled in the art will readily appreciate that the present invention is well adapted to carry out the objects and obtain the ends and advantages mentioned, as well as those inherent therein.

## Claims

1. A vitamin E based anti-cancer compound selected from the group consisting of
a) a compound having the structural formula 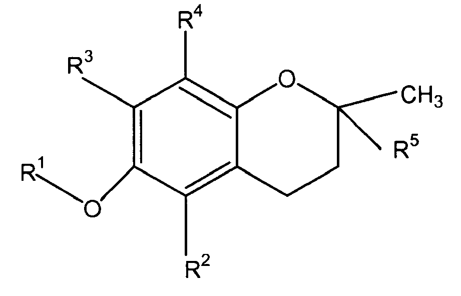 wherein R¹ is a carboxylic acid;
R² and R³ are hydrogen or R⁴;
R⁴ is methyl; and
R⁵ is alkyl;
b) α-tocotrienol;
c) β-tocotrienols;
d) γ-tocotrienol; and
e) δ-tocotrienol,
for use in the treatment of a cell proliferative disease, wherein said compound is to be administered by aerosol delivery of a composition comprising a liposome comprising said compound and a lipid.

2. The vitamin E based compound for use according to claim 1, wherein said compound is 2,5,7,8-tetramethyl-(2R-(4R,8R,12-trimethyltridecyl) chroman-6-yloxy) acetic acid.

3. The vitamin E based compound for use according to claim 1, wherein said compound is α-tocotrienol.

4. The vitamin E based compound for use according to claim 1, wherein said compound is β-tocotrienols.

5. The vitamin E based compound for use according to claim 1, wherein said compound is γ-tocotrienol.

6. The vitamin E based compound for use according to claim 1, wherein said compound is δ-tocotrienol.

7. The vitamin E based compound for use according to any of claims 1 to 6, wherein the lipid is 1,2-dilauroyl-sn-glycero-3-phosphocholine.

8. The vitamin E based compound for use according to any of claims 1 to 7, wherein the ratio of vitamin E based compound to lipid is about 1:3 wt:wt.

9. The vitamin E based compound for use according to any one of claims 1 to 8, wherein the final concentration of said compound in said liposome is no greater than 20.0 mg/ml.

10. The vitamin E based compound for use according to any one of claims 1 to 9, wherein the liposome further comprises a second anti-cancer drug.

11. The vitamin E based compound for use according to claim 10, wherein said anti-cancer drug is 9-nitrocamptothecin, cisplatin, paclitaxel, doxorubicin or celecoxib.

12. Use of a vitamin E based anti-cancer compound selected from the group consisting of
a) a compound having the structural formula 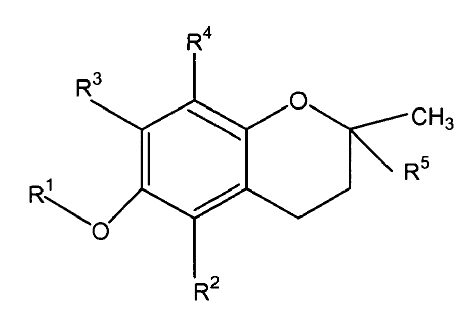 wherein R¹ is a carboxylic acid;
R² and R³ are hydrogen or R⁴;
R⁴ is methyl; and
R⁵ is alkyl;
b) α-tocotrienol;
c) β-tocotrienols;
d) γ-tocotrienol; and
e) δ-tocotrienol,
in the preparation of a medicament for the treatment of a cell proliferative disease in an individual, wherein said compound is to be administered by aerosol delivery of a composition comprising a liposome comprising said compound and a lipid.

13. Use according to claim 12, wherein said treatment further comprises administration of a second anti-cancer drug contained within a delivery vehicle, which is administered in combination or sequentially with said vitamin E based anti-cancer compound.

14. Use according to claim 13, wherein the vitamin E based anti-cancer compound and the second anti-cancer drug are administered in combination by aerosol delivery of a composition comprising a liposome comprising said vitamin E based anti-cancer compound and said second anti-cancer drug.

15. Use according to claim 13 or claim 14, wherein said second anti-cancer drug is 9-nitrocamptothecin, cisplatin, paclitaxel, doxorubicin or celecoxib.

16. Use according to any one of claims 12 to 15, wherein said aerosol delivery is via aerosol nebulization, or an aerosol inhaler.

17. Use according to claim 16, wherein said aerosol nebulization is via a jet nebulizer.

18. Use according to any one of claims 12 to 17, wherein an anti-proliferative effect against the cell proliferative disease is determined via quantitative analysis, qualitative analysis or a combination thereof by detecting a biomarker or by an immunohistochemical assay.

19. Use according to claim 18, wherein said biomarker is KI-67.

20. Use according to either claim 18 or claim 19, wherein said anti-proliferative effect comprises apoptosis, DNA synthesis arrest, cell cycle arrest or cellular differentiation.

21. Use according to any one of claims 12 to 20, wherein said cell proliferative disease is a neoplastic disease or a non-neoplastic disorder.

22. Use according to claim 21, wherein said neoplastic disease is ovarian cancer, cervical cancer, endometrial cancer, bladder cancer, lung cancer, breast cancer, testicular cancer, prostate cancer, gliomas, fibrosarcomas, retinoblastomas, melanomas, soft tissue sarcomas, osteosarcomas, leukemias, colon cancer, carcinoma of the kidney, pancreatic cancer, basal cell carcinoma or squamous cell carcinoma.

23. Use according to claim 21, wherein said non-neoplastic disorder is psoriasis, benign proliferative skin diseases, ichthyosis, papilloma, restenosis, scleroderma, hemangioma, leukoplakia, diseases that develop due to viral infections and disorders having an immune component.

24. Use according to claim 23, wherein said non-neoplastic disorder is a disease that develops due to viral infection or an autoimmune disease.

25. Use according to claim 24, wherein said autoimmune disease is selected from the group consisting of autoimmune thyroiditis, multiple sclerosis, myasthenia gravis, systemic lupus erythematosus, dermatitis herpetiformis, celiac disease and rheumatoid arthritis.

26. Use according to claim 24, wherein said viral infection is Human Immunodeficiency Virus.

27. Use according to claim 23, wherein said disorder having an immune component is the inflammatory process involved in cardiovascular plaque formation or ultraviolet radiation induced skin damage.

## Patentansprüche

1. Auf Vitamin E basierende Antikrebsverbindung, die aus der Gruppe ausgewählt ist, die aus Folgenden besteht:
a) einer Verbindung mit der Strukturformel 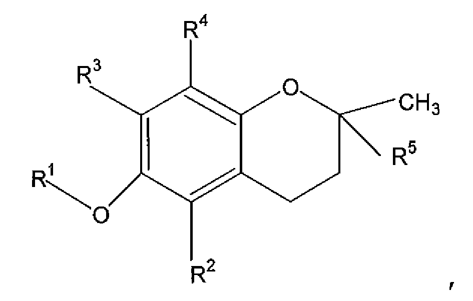 worin R¹ eine Carbonsäure ist;
R² und R³ Wasserstoff oder R⁴ sind;
R⁴ Methyl ist; und
R⁵ Alkyl ist;
b) α-Tocotrienol;
c) β-Tocotrienol;
d) γ-Tocotrienol; und
e) δ-Tocotrienol,
zur Verwendung bei der Behandlung einer zellproliferativen Erkrankung, worin genannte Verbindung durch Aerosolabgabe einer Zusammensetzung, die ein Liposom umfasst, das genannte Verbindung und ein Lipid umfasst, zu verabreichen ist.

2. Auf Vitamin E basierende Verbindung zur Verwendung nach Anspruch 1, worin genannte Verbindung 2,5,7,8-Tetramethyl-(2R-(4R,8R,12-trimethyltridecyl)chroman-6-yloxy)essigsäure ist.

3. Auf Vitamin E basierende Verbindung zur Verwendung nach Anspruch 1, worin genannte Verbindung α-Tocotrienol ist.

4. Auf Vitamin E basierende Verbindung zur Verwendung nach Anspruch 1, worin genannte Verbindung β-Tocotrienol ist.

5. Auf Vitamin E basierende Verbindung zur Verwendung nach Anspruch 1, worin genannte Verbindung γ-Tocotrienol ist.

6. Auf Vitamin E basierende Verbindung zur Verwendung nach Anspruch 1, worin genannte Verbindung δ-Tocotrienol ist.

7. Auf Vitamin E basierende Verbindung zur Verwendung nach einem der Ansprüche 1 bis 6, worin das Lipid 1,2-Dilauroyl-sn-glycero-3-phosphocholin ist.

8. Auf Vitamin E basierende Verbindung zur Verwendung nach einem der Ansprüche 1 bis 7, worin das Gew. : Gew.-Verhältnis der auf Vitamin E basierenden Verbindung zum Lipid etwa 1 : 3 beträgt.

9. Auf Vitamin E basierende Verbindung zur Verwendung nach einem der Ansprüche 1 bis 8, worin die Endkonzentration von genannter Verbindung in genanntem Liposom nicht größer als 20,0 mg/ml ist.

10. Auf Vitamin E basierende Verbindung zur Verwendung nach einem der Ansprüche 1 bis 9, worin das Liposom weiterhin einen zweiten Antikrebswirkstoff umfasst.

11. Auf Vitamin E basierende Verbindung zur Verwendung nach Anspruch 10, worin genannter Antikrebswirkstoff 9-Nitrocamptothecin, Cisplatin, Paclitaxel, Doxorubicin oder Celecoxib ist.

12. Verwendung einer auf Vitamin E basierenden Antikrebsverbindung, die aus der Gruppe ausgewählt ist, die aus Folgenden besteht:
a) einer Verbindung mit der Strukturformel 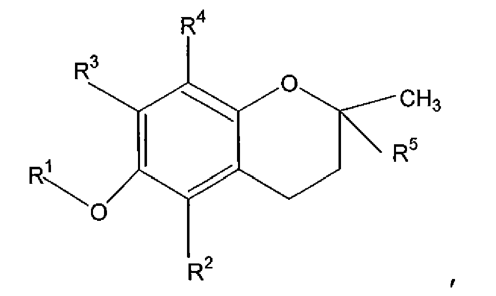 worin R¹ eine Carbonsäure ist;
R² und R³ Wasserstoff oder R⁴ sind;
R⁴ Methyl ist; und
R⁵ Alkyl ist;
c) α-Tocotrienol;
c) β-Tocotrienol;
d) γ-Tocotrienol; und
e) δ-tocotrienol,
bei der Herstellung eines Medikaments zur Behandlung einer zellproliferativen Erkrankung in einem Individuum, worin genannte Verbindung durch Aerosolabgabe einer Zusammensetzung, die ein Liposom umfasst, das genannte Verbindung und ein Lipid umfasst, zu verabreichen ist.

13. Verwendung nach Anspruch 12, worin genannte Behandlung weiterhin die Verabreichung eines zweiten Antikrebswirkstoffes, der in einem Abgabevehikel enthalten ist, das zusammen oder sequentiell mit genannter auf Vitamin E basierender Antikrebsverbindung verabreicht wird, umfasst.

14. Verwendung nach Anspruch 13, worin die auf Vitamin E basierende Antikrebsverbindung und der zweite Antikrebswirkstoff zusammen durch Aerosolabgabe einer Zusammensetzung, die ein Liposom umfasst, das genannte auf Vitamin E basierende Antikrebsverbindung und genannten zweiten Antikrebswirkstoff umfasst, verabreicht werden.

15. Verwendung nach Anspruch 13 oder Anspruch 14, worin genannter zweiter Antikrebswirkstoff 9-Nitrocamptothecin, Cisplatin, Paclitaxel, Doxorubicin oder Celecoxib ist.

16. Verwendung nach einem der Ansprüche 12 bis 15, worin genannte Aerosolabgabe über Aerosolverneblung oder einen Aerosol-Inhalator erfolgt.

17. Verwendung nach Anspruch 16, worin genannte Aerosolverneblung über einen Jet-Vernebler erfolgt.

18. Verwendung nach einem der Ansprüche 12 bis 17, worin eine antiproliferative Wirkung gegen die zellproliferative Erkrankung über quantitative Analyse, qualitative Analyse oder eine Kombination davon durch Nachweisen eines Biomarkers oder durch einen immunohistochemischen Assay bestimmt wird.

19. Verwendung nach Anspruch 18, worin genannter Biomarker KI-67 ist.

20. Verwendung nach entweder Anspruch 18 oder Anspruch 19, worin genannte antiproliferative Wirkung Apoptose, Hemmung der DNA-Synthese, Arretierung des Zellzyklus oder Zelldifferenzierung umfasst.

21. Verwendung nach einem der Ansprüche 12 bis 20, worin genannte zellproliferative Erkrankung eine neoplastische Erkrankung oder eine nicht-neoplastische Störung ist.

22. Verwendung nach Anspruch 21, worin genannte neoplastische Erkrankung Folgendes ist:
Eierstockkrebs, Gebärmutterhalskrebs,
Endometriumkrebs, Blasenkrebs, Lungenkrebs,
Brustkrebs, Hodenkrebs, Prostatakrebs, Gliome,
Fibrosarkome, Retinoblastome, Melanome,
Weichteilsarkome, Osteosarkome, Leukämien, Kolonkrebs,
Karzinom der Niere, Bauchspeicheldrüsenkrebs,
Basalzellkarzinom oder Plattenepithelkarzinom.

23. Verwendung nach Anspruch 21, worin genannte nicht-neoplastische Störung Folgendes ist: Psoriasis, benigne proliferative Hauterkrankungen, Ichthyose, Papillom, Restenose, Sklerodermie, Hämangiom, Leukoplakie, Erkrankungen, die durch virale Infektionen entstehen, und Störungen mit einer Immunkomponente.

24. Verwendung nach Anspruch 23, worin genannte nicht-neoplastische Störung eine Erkrankung, die sich durch virale Infektion entwickelt, oder eine Autoimmunerkrankung ist.

25. Verwendung nach Anspruch 24, worin genannte Autoimmunerkrankung aus der Gruppe ausgewählt ist, die aus Folgenden besteht: Autoimmunthyreoiditis, Multipler Sklerose, Myasthenia gravis, systemischem Lupus erythematodes, Dermatitis herpetiformis, Zöliakie und rheumatoider Arthritis.

26. Verwendung nach Anspruch 24, worin genannte virale Infektion der Human Immunodeficiency Virus (menschlicher Immunschwächevirus) ist.

27. Verwendung nach Anspruch 23, worin genannte Störung mit einer Immunkomponente der inflammatorische Prozess, der bei kardiovaskulärer Plaquebildung beteiligt ist, oder die durch ultraviolette Strahlung induzierte Hautschädigung ist.

## Revendications

1. Composé anti-cancéreux à base de vitamine E sélectionné dans le groupe constitué de
a) un composé ayant la formule structurelle 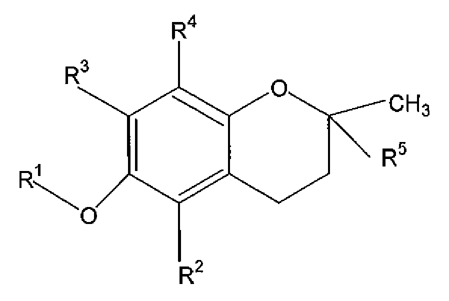 dans laquelle R¹ est un acide carboxylique ;
R² et R³ sont hydrogène ou R⁴ ;
R⁴ est méthyle ; et
R⁵ est alkyle ;
b) le α-tocotriénol ;
c) le β-tocotriénol ;
d) le γ-tocotriénol ; et
e) le δ-tocotriénol,
destiné à être utilisé dans le traitement d'une maladie proliférative cellulaire, dans lequel ledit composé doit être administré par la fourniture en aérosol d'une composition comprenant un liposome comprenant ledit composé et un lipide.

2. Composé à base de vitamine E destiné à être utilisé selon la revendication 1, dans lequel ledit composé est l'acide 2,5,7,8-tétraméthyl-(2R-(4R,8R,12-triméthyltridécyl)chroman-6-yloxy)acétique.

3. Composé à base de vitamine E destiné à être utilisé selon la revendication 1, dans lequel ledit composé est le α-tocotriénol.

4. Composé à base de vitamine E destiné à être utilisé selon la revendication 1, dans lequel ledit composé est le β-tocotriénol.

5. Composé à base de vitamine E destiné à être utilisé selon la revendication 1, dans lequel ledit composé est le γ-tocotriénol.

6. Composé à base de vitamine E destiné à être utilisé selon la revendication 1, dans lequel ledit composé est le δ-tocotriénol.

7. Composé à base de vitamine E destiné à être utilisé selon l'une quelconque des revendications 1 à 6, dans lequel le lipide est la 1,2-dilauroyl-sn-glycéro-3-phosphocholine.

8. Composé à base de vitamine E destiné à être utilisé selon l'une quelconque des revendications 1 à 7, dans lequel le rapport entre composé à base de vitamine E et lipide est d'environ 1/3 en poids/poids.

9. Composé à base de vitamine E destiné à être utilisé selon l'une quelconque des revendications 1 à 8, dans lequel la concentration finale dudit composé dans ledit liposome ne dépasse pas 20,0 mg/ml.

10. Composé à base de vitamine E destiné à être utilisé selon l'une quelconque des revendications 1 à 9, dans lequel le liposome comprend en outre un second médicament anti-cancéreux.

11. Composé à base de vitamine E destiné à être utilisé selon la revendication 10, dans lequel ledit médicament anti-cancéreux est la 9-nitrocamptothécine, le cisplatine, le paclitaxel, la doxorubicine ou le célécoxib.

12. Utilisation d'un composé anti-cancéreux à base de vitamine E sélectionné dans le groupe constitué de
a) un composé ayant la formule structurelle 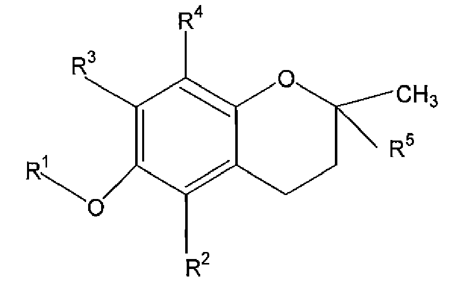 dans laquelle R¹ est un acide carboxylique ;
R² et R³ sont hydrogène ou R⁴ ;
R⁴ est éthyle et
R⁵ est alkyle ;
b) le α-tocotriénol
c) le β-tocotriénol ;
d) le γ-tocotriénol ; et
e) le δ-tocotriénol,
dans la préparation d'un médicament pour le traitement d'une maladie proliférative cellulaire chez un individu, dans laquelle ledit composé doit être administré par la fourniture en aérosol d'une composition comprenant un liposome comprenant ledit composé et un lipide.

13. Utilisation selon la revendication 12, dans laquelle ledit traitement comprend en outre l'administration d'un second médicament anti-cancéreux contenu au sein d'un excipient de fourniture, qui est administré en combinaison ou de manière séquentielle avec ledit composé anti-cancéreux à base de vitamine E.

14. Utilisation selon la revendication 13, dans laquelle le composé anti-cancéreux à base de vitamine E et le second médicament anti-cancéreux sont administrés en combinaison par fourniture en aérosol d'une composition comprenant un liposome comprenant ledit composé anti-cancéreux à base de vitamine E et ledit second médicament anti-cancéreux.

15. Utilisation selon la revendication 13 ou la revendication 14, dans laquelle ledit second médicament anti-cancéreux est la 9-nitrocamptothécine, le cisplatine, le paclitaxel, la doxorubicine ou le célécoxib.

16. Utilisation selon l'une quelconque des revendications 12 à 15, dans laquelle ladite fourniture en aérosol est par le biais d'une nébulisation par aérosol ou d'un inhalateur par aérosol.

17. Utilisation selon la revendication 16, dans laquelle ladite nébulisation par aérosol est par le biais d'un nébulisateur à jet.

18. Utilisation selon l'une quelconque des revendications 12 à 17, dans laquelle un effet anti-prolifératif contre la maladie proliférative cellulaire est déterminé par le biais d'une analyse quantitative, d'une analyse qualitative ou d'une combinaison de celles-ci en détectant un biomarqueur ou par un dosage immunohistochimique.

19. Utilisation selon la revendication 18, dans laquelle ledit biomarqueur est KI-67.

20. Utilisation selon la revendication 18 ou la revendication 19, dans laquelle ledit effet anti-prolifératif comprend l'apoptose, l'arrêt de la synthèse d'ADN, l'arrêt du cycle cellulaire ou la différenciation cellulaire.

21. Utilisation selon l'une quelconque des revendications 12 à 20, dans laquelle ladite maladie proliférative cellulaire est une maladie néoplasique ou un trouble non néoplasique.

22. Utilisation selon la revendication 21, dans laquelle ladite maladie néoplasique est le cancer ovarien, le cancer cervical, le cancer endométrial, le cancer de la vessie, le cancer du poumon, le cancer du sein, le cancer testiculaire, le cancer de la prostate, des gliomes, des fibrosarcomes, des rétinoblastomes, des mélanomes, des sarcomes du tissu mou, des ostéosarcomes, des leucémies, le cancer du côlon, le carcinome du rein, le cancer pancréatique, le carcinome à cellules basales ou le carcinome à cellules squameuses.

23. Utilisation selon la revendication 21, dans laquelle ledit trouble non néoplasique est le psoriasis, des maladies cutanées prolifératives bénignes, l'ichthyose, le papillome, la resténose, la sclérodermie, l'hémangiome, la leukoplasie, des maladies qui se développent en raison d'infections virales et des troubles ayant un composant immunitaire.

24. Utilisation selon la revendication 23, dans laquelle ledit trouble non néoplasique est une maladie qui se développe en raison d'une infection virale ou une maladie auto-immune.

25. Utilisation selon la revendication 24, dans laquelle ladite maladie auto-immune est sélectionnée dans le groupe constitué de la thyroïdite auto-immune, la sclérose en plaques, la myasthénie grave, le lupus érythémateux systémique, la dermatite herpétiforme, la maladie coeliaque et la polyarthrite rhumatoïde.

26. Utilisation selon la revendication 24, dans laquelle ladite infection virale est le virus de l'immunodéficience humaine.

27. Utilisation selon la revendication 23, dans laquelle ledit trouble ayant un composant immunitaire est le processus inflammatoire impliqué dans la formation de plaques cardiovasculaires ou l'endommagement cutané induit par un rayonnement ultraviolet.
